# EUROPEAN PATENT SPECIFICATION

(11) **EP 3 326 394 B1**
(45) Date of publication and mention of the grant of the patent: **01.09.2021**
(21) Application number: 16831062.1
(22) Date of filing: 19.07.2016
(51) Int. Cl.: H04W 4/02, H04W 84/12, G06Q 10/08, H04W 4/029, H04W 4/80

(54) **WIRELESS BRIDGE HARDWARE SYSTEM FOR ACTIVE RFID IDENTIFICATION AND LOCATION TRACKING**
DRAHTLOSBRÜCKEN-HARDWARESYSTEM FÜR AKTIVE RFID-IDENTIFIZIERUNG UND STANDORTVERFOLGUNG
SYSTÈME MATÉRIEL DE PONT SANS FIL PERMETTANT L'IDENTIFICATION PAR RADIO-FRÉQUENCE ACTIVE (RFID) ET DE SUIVI DE POSITION

(30) Priority: 24.07.2015 US 201514808624
(43) Date of publication of application: 30.05.2018
(73) Proprietor: General Electric Company, Schenectady, NY 12345 (US)
(72) Inventor: THEURER, Charles, Niskayuna, New York 12309 (US); GOOD, Brandon, Niskayuna, New York 12309 (US); LIU, Shaopeng, Niskayuna, New York 12309 (US)
(74) Representative: Fennell, Gareth Charles
(86) International application number: PCT/US2016/042946
(87) International publication number: WO 2017/019383

(56) References cited:
- WO-A1-00/60374
- WO-A1-2015/097314
- US-A1- 2006 261 951
- US-A1- 2006 261 951
- US-A1- 2010 090 901
- US-A1- 2014 327 521
- US-A1- 2015 105 099
- US-B2- 7 312 752

## Description

### TECHNICAL FIELD

Embodiments of the present disclosure relate generally to radio frequency identification and, more particularly, but not by way of limitation, to integrating radio frequency identification with a wireless network.

### BACKGROUND

Radio-frequency identification (RFID) is the wireless use of electromagnetic fields to transfer data, for the purposes of automatically identifying and tracking tags attached to objects. The tags contain electronically stored information. An RFID reader includes a receiver that can decode the electronically stored information. Some tags are powered by electromagnetic induction from magnetic fields produced near the reader. Some types collect energy from the interrogating radio waves and act as a passive transponder. Other types of tags have a local power source such as a battery and may operate at hundreds of meters from the reader.

RFID tags are used in a variety of environments, such as in health care, retail or commercial spaces, and industrial warehouses. In a healthcare environment, such as a hospital or clinic, the environment can include a variety of information systems, such as hospital information systems (HIS), radiology information systems (RIS), clinical information systems (CIS), and cardiovascular information systems (CVIS), and storage systems, such as picture archiving and communication systems (PACS), library information systems (LIS), and electronic medical records (EMR). These systems may store a variety of information such as patient medication orders, medical histories, imaging data, test results, diagnosis information, management information, and/or scheduling information. A retail or commercial space can also include a variety of information systems, such as a point-of-sale system, a payment information processing system, an inventory management system, and other such systems. These systems may also store a variety of information such as inventory types and amounts, how long a given inventory item has been displayed, how often a given inventory item has been sold, and other such information.

The effectiveness of a healthcare system can be determined by the interaction between the healthcare resources. Adverse events that occur in hospitals, such as hospital-acquired infections, lost or missing assets, etc., result in patient harm, increased recovery time, loss of a hospital's and its staffs capacity to serve, unreimbursed healthcare costs, and, generally, increased healthcare costs. One of the main causes of these events is non-adherence to protocols. Protocols can refer to a series of preferred or prescribed tasks that (1) have been proven to reduce adverse events and (2) effect a desired elimination of activities, practices, or patterns that create harm or inefficiency. Example uses of such protocols are for hand washing, fall prevention, rounding, pain management, sleep improvement and physical therapy.

Similarly, the effectiveness of a retail space can be determined by how often inventory items sell or whether a given portion of the retail space is being frequented by customers. Increases in customer visits to a particular location in a store may indicate that customers have an interest in items being sold in that particular location, while decreases in customer visits to other parts of a store may indicate that customers are no longer interested in the items being sold in that part of the store. Thus, locations in a store experiencing a decrease in customer visits impact profitability as the items in those locations are less likely to be sold.

US2014/327521 A1 describes a method for asset location via a relay tag which includes receiving, at the relay tag, an asset message from an asset tag according to a wireless communication protocol, wherein the relay tag has a known location; producing, at the relay tag, a relay message using information contained in the asset message, wherein the relay message includes an indication of the relay tag from which the relay message is sent and an indication of the asset tag from which the asset message was received; and sending the relay message from the relay tag to a receiver using the wireless communication protocol.

US2006261951 A1 describes a series of radio frequency identification (RFID) systems . One such RFID system comprises an RFID signpost, and a plurality of infrared transmitters, wherein each infrared transmitter of the plurality of infrared transmitters is arranged to cover, when transmitting, a distinct sector relative to the RFID signpost. Another RFID system comprises an RFID signpost having a transmitter for transmitting signals of a predefined type, and a receiver for receiving signals of the predefined type, wherein the transmitter for transmitting signals of the predefined type cannot transmit until a determination is made that the predefined type of signal is not present at the receiver. Still another RFID system comprises an RFID signpost including a transmitter having a continuous power dissipation rating, and a processor for controlling the transmitter such that peak pulse power of a transmission from the transmitter multiplied by its duty cycle does not exceed the continuous power dissipation rating for the transmitter.

US2010090901 describes techniques for accurate position location and tracking suitable for a wide range of facilities invariable environments are disclosed. In one aspect, a system for position location comprises a plurality of sensors (e.g. a network monitor, an environment sensor) for generating measurements of a plurality of sources, a plurality of objects or tags, each object generating measurements of the plurality of sources, and a processor for receiving the measurements and generating a position location for one or more objects in accordance with the received measurements. In another aspect, a position engine comprises a mapped space of a physical environment, and a processor for updating the mapped space in response to received measurements. The position engine may receive second measurements from an object within the physical environment, and generate a position location estimate for the object from the received second measurements and the mapped space.

### SUMMARY

Aspects of the present invention are defined in the appended independent claims. Dependent claims constitute embodiments of the invention. Examples not covered by the amended claims are provided as background useful for understanding the invention.

### BRIEF DESCRIPTION OF THE DRAWINGS

Various ones of the appended drawings merely illustrate example embodiments of the present disclosure and cannot be considered as limiting its scope.
**FIG.** 1 is a block diagram of an information system according to an example embodiment.
**FIG.** 2 is a block diagram of an information infrastructure according to an example embodiment.
**FIG.** 3 is an industrial Internet configuration according to an example embodiment.
**FIG.** 4 is a block diagram illustrating an environment that facilitates proximity detection and location tracking using a mobile wireless bridge, according to an example embodiment.
**FIG.** 5 illustrates various components included in a beacon tag, a beacon badge, as hub module and a dock module, according to various example embodiments.
**FIG.** 6 illustrates a beacon badge, according to an example embodiment.
**FIGS. 7A-7B** illustrate additional details of the beacon badge, according to an example embodiment.
**FIG.** 8 illustrates alternative implementations to the beacon badge of FIG. 6, according to further embodiments.
**FIG.** 9 illustrates an example environment to facilitate proximity detection and location tracking using a mobile wireless bridge, according to an example embodiment.
**FIG. 10** illustrates another example environment to facilitate proximity detection and location tracking using a mobile wireless bridge, according to another example embodiment.
**FIG.** 11 illustrates yet a further environment to facilitate proximity detection and location tracking using a mobile wireless bridge, according to a further embodiment.
**FIG. 12** illustrates another environment to facilitate proximity detection and location tracking using a mobile wireless bridge, according to another embodiment.
**FIG.** 13 illustrates a method for operating the disclosed wireless bridge according to an example embodiment.
**FIG. 14** is graph correlating average RSSI values with distance based on the angle at a beacon badge receives a signal from a beacon tag.
**FIG. 15** illustrates a diagrammatic representation of a machine in the form of a computer system within which a set of instructions may be executed for causing the machine to perform any one or more of the methodologies discussed herein, according to an example embodiment.
The headings provided herein are merely for convenience and do not necessarily affect the scope or meaning of the terms used.

### DETAILED DESCRIPTION

The description that follows includes systems, methods, techniques, instruction sequences, and computing machine program products that embody illustrative embodiments of the disclosure. In the following description, for the purposes of explanation, numerous specific details are set forth in order to provide an understanding of various embodiments of the inventive subject matter. It will be evident, however, to those skilled in the art, that embodiments of the inventive subject matter may be practiced without these specific details. In general, well-known instruction instances, protocols, structures, and techniques are not necessarily shown in detail.

Certain examples of the disclosed technology improve proximity detection and location tracking with a mobile wireless bridge. The disclosed systems and methods include one or more beacon tags positioned within an environment, such as a hospital room, retail space, industrial environment, or other such environment, and that transmit beacon messages. In one embodiment, the beacon messages are transmitted at fixed time intervals. In alternative embodiments, the beacon messages are transmitted at variable time intervals or as a single instance.

The beacon messages are received by a mobile wireless bridge platform that listens for low power Bluetooth Low Energy (BLE) beacon messages and connects to a Wi-Fi infrastructure. The mobile wireless bridge platform (sometimes referred to herein as a "badge" or "beacon badge") processes the received beacon messages and communicates information obtained from the beacon messages to a real-time location services (RTLS) server via the Wi-Fi infrastructure (e.g., a wireless network). The RTLS server aggregates the information received from one or more badges over an interval of time and reports the aggregated information. In various embodiments, the reports may include text and/or graphics, such as charts, graphs, tables, etc.

The disclosed real-time location services can improve the efficiency of a working environment, such as patient workflow in a hospital environment, through proximity detection and location tracking. Location tracking can be used to locate assets (e.g., intravenous (IV) pumps, telemetry units, wheelchairs, etc.) within the hospital. For example, location tracking can be used to locate a "lost" or "missing" IV pump within a patient's room. Proximity detection facilitates an improved understanding of how interactions occur during the patient workflow. For example, based on the proximity to a soap dispenser, a user (e.g., a system administrator) can determine whether a caretaker washed his or her hands prior to interacting with a patient.

In another environment, such as a retail space, the disclosed real-time services can improve profitability and customer interactions by monitoring the sale of inventory and locations that customers tend to visit more frequently. For example, based on whether a location in a store is visited more or less frequently, the owner of the store can make adjustments in the inventory that are located at the location, such as by changing the inventory at the location or by increasing the amount of inventory at the location.

The technical effect of the disclosed RTLS system is that it facilitates improved proximity detection and location tracking by creating a tracking network within an environment using an existing network infrastructure, such as a wireless network infrastructure. Beacon tags are installed throughout a location or building. For example, beacon tags can be affixed to stationary and mobile assets such as hospital beds, IV pumps, soap dispensers, clothing, toys, kitchenwares, automobile accessories, industrial tools, or other such items. Beacon tags may also be included in disposable patient tags provided to the patient upon admission of a hospital stay.

Beacon tags are low-cost, low-power transmitters of beacons. A beacon (sometimes referred to herein as a "beacon message") includes information about the beacon tag such as a unique identifier and location information. In one embodiment, the beacon tags broadcast beacon messages at fixed time intervals. In alternative embodiments, the beacon messages are transmitted at variable time intervals or as a single instance.

Beacon badges are mobile wireless bridges that facilitate mobile tracking by "listening" and receiving the beacon messages. The beacon badge includes a BLE chip to receive connection-less beacon messages from beacon tags and a wireless transceiver to establish a connection with an RTLS server. The beacon badges may be worn or transported by an employee, such as a doctor, retail clerk, equipment operator, or other such employee. As the employee moves about the environment, the beacon badge passively collects beacon messages and communicates beacon packets to an RTLS server at the backend of the system.

In some examples, the beacon badge collects a number (e.g., a predetermined number) of beacon messages or waits a given amount of time prior to communicating the beacon packets. In some examples, the beacon badge communicates a beacon packet as a beacon message is received. A beacon packet includes information received from the beacon message such as a unique identifier of the source beacon tag and a spatial location of the source beacon tag. The beacon badge also includes in the beacon packet a timestamp identifying when the beacon message was received by the beacon badge and a received signal strength indication (RSSI) value (e.g., a power ratio in decibels of the measured power to one milli-watt (dBm)).

Example beacon badges disclosed herein include a proximity engine to process the beacon messages and determine a distance from the source of the beacon messages (e.g., the beacon tag that broadcast the corresponding beacon message). For example, a hospital room may include a first beacon tag affixed to a door, a second beacon tag affixed to an infusion pump, a third beacon tag affixed to a bed, and a fourth beacon tag included in a patient tag (e.g., a disposable bracelet including patient identification information such as name, sex, date of birth information). As the caregiver moves about the hospital room, the beacon badge may receive beacon messages from each of the beacon tags. The proximity tag can determine the RSSI value for each of the beacon messages and associate an RSSI value with a respective beacon tag.

In some examples, the proximity engine determines which beacon tags are proximate (e.g., near or closely located) to the beacon badge. For example, the proximity engine can compare the RSSI value of a beacon message to a threshold and if the RSSI value satisfies the threshold (e.g., the RSSI value is greater than a threshold value), the proximity engine identifies the source beacon tag as proximate to the beacon badge. In some examples, the proximity engine discards beacon messages that are not proximate to the beacon badge.

Example systems and methods disclosed herein include an RTLS server that monitors and/or reports tracking location and interactions between people and assets in an environment. For example, the RTLS server aggregates beacon packets from the one or more beacon badges included in the environment (e.g., the hospital). The RTLS server communicates with the beacon badges via a wireless network, such as a wireless local area network, a wireless wide area network, or a combination thereof.

In one embodiment, the disclosed wireless network bridge, beacon badges, and beacon tags are used in the processing of health information. While the following description is directed to the processing of health information, one of ordinary skill in the art will recognize that the described systems and methods can be adapted for other environments and/or infrastructures. Thus, the following description of employing the disclosed systems and methods in the processing of health information, such description should be understood as not excluding other applications of such systems and methods.

Health information, also referred to as healthcare information and/or healthcare data, relates to information generated and/or used by a healthcare entity. Health information can be information associated with health of one or more patients, for example. Health information may include protected health information (PHI), as outlined in the Health Insurance Portability and Accountability Act (HIPAA), which is identifiable as associated with a particular patient and is protected from unauthorized disclosure. Health information can be organized as internal information and external information. Internal information includes patient encounter information (e.g., patient-specific data, aggregate data, comparative data, etc.) and general healthcare operations information, etc. External information includes comparative data, expert and/or knowledge-based data, etc. Information can have both a clinical (e.g., diagnosis, treatment, prevention, etc.) and administrative (e.g., scheduling, billing, management, etc.) purpose.

Institutions, such as healthcare institutions, having complex network support environments and sometimes chaotically driven process flows utilize secure handling and safeguarding of the flow of sensitive information (e.g., personal privacy). A need for secure handling and safeguarding of information increases as a demand for flexibility, volume, and speed of exchange of such information grows. For example, healthcare institutions provide enhanced control and safeguarding of the exchange and storage of sensitive patient PHI and employee information between diverse locations to improve hospital operational efficiency in an operational environment typically having a chaotic-driven demand by patients for hospital services. In certain examples, patient-identifying information can be masked or even stripped from certain data depending upon where the data is stored and who has access to that data. In some examples, PHI that has been "de-identified" can be re-identified based on a key and/or other encoder/decoder.

A healthcare information technology infrastructure can be adapted to service multiple business interests while providing clinical information and services. Such an infrastructure may include a centralized capability including, for example, a data repository, reporting, discreet data exchange/connectivity, "smart" algorithms, personalization/consumer decision support, etc. This centralized capability provides information and functionality to a plurality of users including medical devices, electronic records, access portals, pay for performance (P4P), chronic disease models, and clinical health information exchange/regional health information organization (HIE/RHIO), and/or enterprise pharmaceutical studies, home health, for example.

Interconnection of multiple data sources facilitates engagement of all relevant members of a patient's care team and improves the administrative and management burden on the patient for managing his or her care. Particularly, interconnecting the patient's electronic medical record and/or other medical data improves patient care and management of patient information. Furthermore, patient care compliance is facilitated by providing tools that automatically adapt to the specific and changing health conditions of the patient and provide comprehensive education and compliance tools to drive positive health outcomes.

In certain examples, healthcare information can be distributed among multiple applications using a variety of database and storage technologies and data formats. To provide a common interface and access to data residing across these applications, a connectivity framework (CF) can be provided which leverages common data and service models (CDM and CSM) and service oriented technologies, such as an enterprise service bus (ESB) to provide access to the data.

In certain examples, a variety of user interface frameworks and technologies can be used to build applications for health information systems including, but not limited to, MICROSOFT® ASP.NET, AJAX®, MICROSOFT® Windows Presentation Foundation, GOOGLE® Web Toolkit, MICROSOFT® Silverlight, ADOBE®, and others. Applications can be composed from libraries of information widgets to display multi-content and multi-media information, for example. In addition, the framework enables users to tailor layout of applications and interact with underlying data.

In certain examples, an advanced Service-Oriented Architecture (SOA) with a modern technology stack provides robust interoperability, reliability, and performance. An example SOA can include a three-fold interoperability strategy having a central repository (e.g., a central repository built from Health Level Seven (HL7) transactions), services for working in federated environments, and visual integration with third-party applications. Certain examples provide portable content enabling "plug 'n play" content exchange among healthcare organizations. A standardized vocabulary using common standards (e.g., LOINC, SNOMED CT, RxNorm, FDB, ICD-9, ICD-10, etc.) is used for interoperability, for example. Certain examples provide an intuitive user interface to help minimize end-user training. Certain examples facilitate user-initiated launching of third-party applications directly from a desktop interface to help provide a seamless workflow by sharing user, patient, and/or other contexts. Certain examples provide real-time (or at least substantially real time assuming some system delay) patient data from one or more information technology (IT) systems and facilitate comparison(s) against evidence-based best practices. Certain examples provide one or more dashboards for specific sets of patients. Dashboard(s) can be based on condition, role, and/or other criteria to indicate variation(s) from a desired practice, for example.

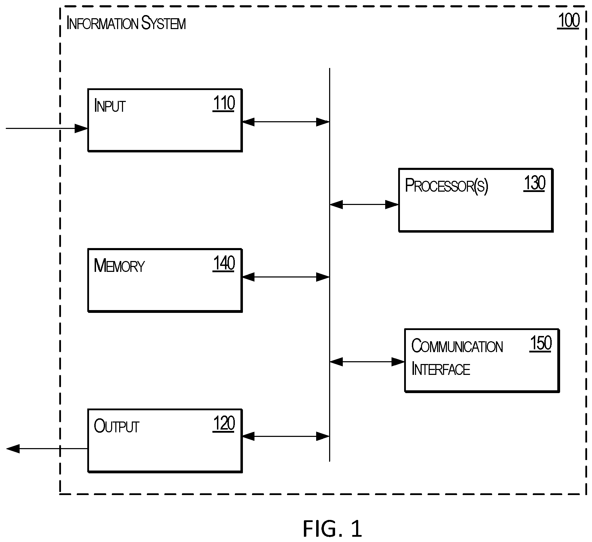

Referring to **FIG. 1** is a block diagram of an information system **100** according to an example embodiment. Where the information system **100** is implemented within a healthcare organization, the information system **100** incorporates a variety of systems and processes including, but not limited to, image storage (e.g., picture archiving and communication system (PACS), etc.), image processing and/or analysis, radiology reporting and/or review (e.g., radiology information system (RIS), etc.), computerized provider order entry (CPOE) system, clinical decision support, patient monitoring, population health management (e.g., population health management system (PHMS), health information exchange (HIE), etc.), healthcare data analytics, cloud-based image sharing, electronic medical record (e.g., electronic medical record system (EMR), electronic health record system (EHR), electronic patient record (EPR), personal health record system (PHR), etc.), and/or other health information system (e.g., clinical information system (CIS), hospital information system (HIS), patient data management system (PDMS), laboratory information system (LIS), cardiovascular information system (CVIS), etc.

In other environments, such as a retail storefront or industrial center, the information system **100** includes additional or alternative systems and processes, such as a customer relationship management systems, inventory management systems, point-of-sale systems, record management systems, employee management systems, payroll management systems, quality and document control systems, and other such systems or combination of systems.

As illustrated in **FIG. 1****,** the information system **100** includes an input **110,** an output **120,** one or more processors **130,** a memory **140,** and a communication interface **150.** The components of example system **100** can be integrated in one device or distributed over two or more devices.

The input **110** may include a keyboard, a touch-screen, a mouse, a trackball, a track pad, optical barcode recognition, voice command, etc. or combination thereof used to communicate an instruction or data to system **100.** The input **110** may include an interface between systems, between user(s) and system **100,** etc.

In one embodiment, the output **120** provides a display generated by processor **130** for visual illustration on a monitor or the like. The display can be in the form of a network interface or graphic user interface (GUI) to exchange data, instructions, or illustrations on a computing device via communication interface **150,** for example. Examples of output **120** include a monitor (e.g., liquid crystal display (LCD), plasma display, cathode ray tube (CRT), etc.), light emitting diodes (LEDs), a touch-screen, a printer, a speaker, or other conventional display device or combination thereof.

The one or more processors **130** include hardware and/or software for configuring the hardware to execute one or more tasks and/or implement a particular system configuration. The one or more processors **130** process data received at input **110** and generate a result that can be provided to one or more of output **120,** memory **140,** and/or communication interface **150.** For example, and in one embodiment, the one or more processors **130** receive user annotation provided via input **110** with respect to an image displayed via output **120** and generate a report associated with the image based on the annotation. As another example, the one or more processors **130** process updated patient information obtained via input **110** to provide an updated patient record to an EMR via communication interface **150.** As yet a further example, the one or more processors **130** process inventory quantities received via input **110** and generate a report associated with the received inventory levels via output **120.**

The memory **140** may include a relational database, an object-oriented database, a data dictionary, a clinical data repository, a data warehouse, a data mart, a vendor neutral archive, an enterprise archive, etc. In one embodiment, the memory **140** stores images, patient data, best practices, clinical knowledge, analytics, reports, etc. In another embodiment, the memory **140** stores employee records, inventory types and quantities, payroll information, and other retail information. The memory **140** stores data and/or instructions accessible by the processor **130.** In certain examples, memory **140** is accessible by an external system via the communication interface **150.**

In certain examples, memory **140** stores and controls access to encrypted information, such as patient records, encrypted update-transactions for patient medical records, including usage history, etc. In an example, medical records can be stored without using logic structures specific to medical records. In such a manner, memory **140** stores information securely. For example, a patient's data can be encrypted with a unique patient-owned key at the source of the data. The data is then uploaded to memory **140.** Privacy with respect to the patient data is maintained as the memory **140** may not store unencrypted patient data. The patient's data can be downloaded and decrypted locally with the encryption key.

Where the information system **100** is implemented for healthcare, the memory **140** can be structured according to provider, patient, patient/provider association, and document. Provider information may include, for example, an identifier, a name, and address, a public key, and one or more security categories. Patient information may include, for example, an identifier, a password hash, and an encrypted email address. Patient/provider association information may include a provider identifier, a patient identifier, an encrypted key, and one or more override security categories. Document information may include an identifier, a patient identifier, a clinic identifier, a security category, and encrypted data, for example.

The communication interface **150** facilitates transmission of electronic data within and/or among one or more systems. Communication via communication interface **150** can be implemented using one or more protocols. Where the information system **100** is implemented for healthcare management, communication via communication interface **150** occurs according to one or more standards (e.g., Digital Imaging and Communications in Medicine (DICOM), Health Level Seven (HL7), ANSI X12N, etc.). However, other standards may also be used depending on the context in which the information system **100** is implemented.

The communication interface **150** can be a wired interface (e.g., a data bus, a Universal Serial Bus (USB) connection, etc.) and/or a wireless interface (e.g., radio frequency, infrared, near field communication (NFC), etc.). For example, communication interface **150** may communicate via wired local area network (LAN), wireless LAN, wide area network (WAN), etc. using one or more communication protocols (e.g., BLUETOOTH™, USB 2.0, USB 3.0, etc.) or combination of protocols.

In certain examples, a Web-based portal may be used to facilitate access to information, such as patient care, practice management, employee information, etc. Information and/or functionality available via the Web-based portal may include one or more of order entry, laboratory test results review system, patient information, clinical decision support, medication management, scheduling, electronic mail and/or messaging, medical resources, etc. In certain examples, a browser-based interface can serve as a zero footprint, zero download, and/or other universal viewer for a client device.

In certain examples, the Web-based portal serves as a central interface to access information and applications, for example. Data may be viewed through the Web-based portal or viewer, for example. Additionally, data may be manipulated and propagated using the Web-based portal, for example. Data may be generated, modified, stored and/or used and then communicated to another application or system to be modified, stored and/or used, for example, via the Web-based portal, for example.

The Web-based portal may be accessible locally (e.g., in an office) and/or remotely (e.g., via the Internet and/or other private network or connection), for example. The Web-based portal may be configured to help or guide a user in accessing data and/or functions to facilitate patient care and practice management, for example. In certain examples, the Web-based portal may be configured according to certain rules, preferences and/or functions, for example. For example, a user may customize the Web portal according to particular desires, preferences and/or requirements.

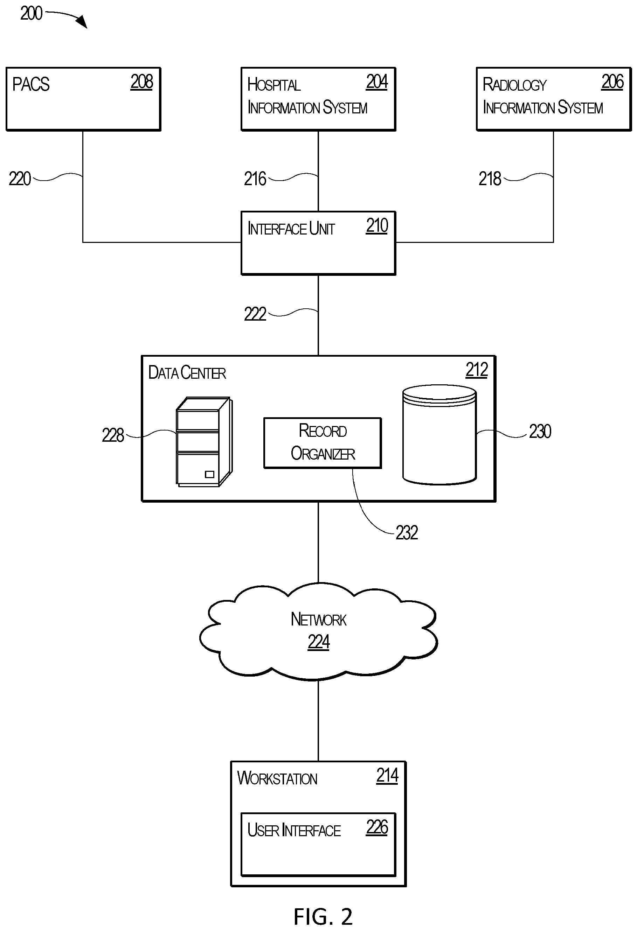

**FIG. 2** is a block diagram of an information infrastructure **100** according to an example embodiment. In one embodiment, the information infrastructure **100** is implemented as a healthcare-oriented system. In this embodiment, the information infrastructure **100** includes an HIS **204,** an RIS **206,** a PACS **208,** an interface unit **210,** a data center **212,** and a workstation **214.**

In one embodiment, HIS **204,** RIS **206,** and PACS **208** are housed in a healthcare facility and locally archived. However, in other implementations, HIS **204,** RIS **206,** and/or PACS **208** may be housed within one or more other suitable locations. In alternative embodiments, one or more of PACS **208,** RIS **206,** HIS **204,** are implemented remotely via a thin client and/or downloadable software solution. Furthermore, one or more components of the healthcare system **200** can be combined and/or implemented together. For example, RIS **206** and/or PACS **208** can be integrated with HIS **204;** PACS **208** can be integrated with RIS **206;** and/or the three example information systems **204, 206,** and/or **208** can be integrated together.

In yet other embodiments, healthcare system **200** includes a subset of the illustrated information systems **204, 206,** and/or **208.** For example, healthcare system **200** may include only one or two of HIS **204,** RIS **206,** and/or PACS **208.** Information (e.g., scheduling, test results, exam image data, observations, diagnosis, etc.) can be entered into HIS **204,** RIS **206,** and/or PACS **208** by healthcare practitioners (e.g., radiologists, physicians, and/or technicians) and/or administrators before and/or after patient examination. One or more of the HIS **204,** RIS **206,** and/or PACS **208** can communicate with equipment and system(s) in an operating room, patient room, etc., to track activity, correlate information, generate reports and/or next actions, and the like.

The HIS **204** stores medical information such as clinical reports, patient information, and/or administrative information received from, for example, personnel at a hospital, clinic, and/or a physician's office (e.g., an EMR, EHR, PHR, etc.). RIS **206** stores information such as, for example, radiology reports, radiology exam image data, messages, warnings, alerts, patient scheduling information, patient demographic data, patient tracking information, and/or physician and patient status monitors. Additionally, RIS **206** enables exam order entry (e.g., ordering an x-ray of a patient) and image and film tracking (e.g., tracking identities of one or more people that have checked out a film). In some examples, information in RIS **206** is formatted according to the HL-7 (Health Level Seven) clinical communication protocol. In certain examples, a medical exam distributor is located in RIS **206** to facilitate distribution of radiology exams to a radiologist workload for review and management of the exam distribution by, for example, an administrator.

PACS **208** stores medical images (e.g., x-rays, scans, three-dimensional renderings, etc.) as, for example, digital images in a database or registry. In some examples, the medical images are stored in PACS **208** using the Digital Imaging and Communications in Medicine (DICOM) format. Images are stored in PACS **208** by healthcare practitioners (e.g., imaging technicians, physicians, radiologists) after a medical imaging of a patient and/or are automatically transmitted from medical imaging devices to PACS **208** for storage. In some examples, PACS **208** can also include a display device and/or viewing workstation to enable a healthcare practitioner or provider to communicate with PACS **208.**

The interface unit **210** includes a hospital information system interface connection **216,** a radiology information system interface connection **218,** a PACS interface connection **220,** and a data center interface connection **222.** Interface unit **210** facilities communication among HIS **204,** RIS **206,** PACS **208,** and/or data center **212.** Interface connections **216, 218, 220,** and **222** can be implemented by, for example, a Wide Area Network (WAN) such as a private network or the Internet. Accordingly, interface unit **210** includes one or more communication components such as, for example, an Ethernet device, an asynchronous transfer mode (ATM) device, an 802.11 device, a DSL modem, a cable modem, a cellular modem, etc. In turn, the data center **212** communicates with workstation **214,** via a network **224,** implemented at a plurality of locations (e.g., a hospital, clinic, doctor's office, other medical office, or terminal, etc.). Network **224** is implemented by, for example, the Internet, an intranet, a private network, a wired or wireless Local Area Network, and/or a wired or wireless Wide Area Network. In some examples, interface unit **210** also includes a broker (e.g., a Mitra Imaging's PACS Broker) to allow medical information and medical images to be transmitted together and stored together.

Interface unit **210** receives images, medical reports, administrative information, exam workload distribution information, and/or other clinical information from the information systems **204, 206, 208** via the interface connections **216, 218, 220.** In some embodiments, such as when different formats of the received information are incompatible, interface unit **210** translates or reformats (e.g., into Structured Query Language ("SQL") or standard text) the medical information, such as medical reports, to be stored at data center **212.** The reformatted medical information can be transmitted using a transmission protocol to enable different medical information to share common identification elements, such as a patient name or social security number. Next, interface unit **210** transmits the medical information to data center **212** via data center interface connection **222.** Finally, medical information is stored in data center **212** in, for example, the DICOM format, which enables medical images and corresponding medical information to be transmitted and stored together.

The medical information is later viewable and retrievable at workstation **214** (e.g., by their common identification element, such as a patient name or record number). Workstation **214** can be any equipment (e.g., a personal computer) capable of executing software that permits electronic data (e.g., medical reports) and/or electronic medical images (e.g., x-rays, ultrasounds, MRI scans, etc.) to be acquired, stored, or transmitted for viewing and operation. Workstation **214** receives commands and/or other input from a user via, for example, a keyboard, mouse, track ball, microphone, etc.

In one embodiment, workstation **214** implements a user interface **226** to enable a healthcare practitioner and/or administrator to interact with healthcare system **200.** For example, in response to a request from a physician, user interface **226** presents a patient medical history. In other examples, a radiologist is able to retrieve and manage a workload of exams distributed for review to the radiologist via user interface **226.** In further examples, an administrator reviews radiologist workloads, exam allocation, and/or operational statistics associated with the distribution of exams via user interface **226.** In some examples, the administrator adjusts one or more settings or outcomes via user interface **226.**

The data center **212** stores information such as images, data, medical reports, and/or, more generally, patient medical records. In addition, data center **212** acts as a central conduit to information located at other sources such as, for example, local archives, hospital information systems/radiology information systems (e.g., HIS **204** and/or RIS **206),** or medical imaging/storage systems (e.g., PACS **208** and/or connected imaging modalities). That is, the data center **212** can store links or indicators (e.g., identification numbers, patient names, or record numbers) to information. In the illustrated example, data center **212** is managed by an application server provider (ASP) and is located in a centralized location that can be accessed by a plurality of systems and facilities (e.g., hospitals, clinics, doctor's offices, other medical offices, and/or terminals). In various embodiments, the data center **212** is spatially distant from HIS **204,** RIS **206,** and/or PACS **208.**

In one embodiment, the data center **212** includes a server **228,** a database **230,** and a record organizer **232.** Server **228** receives, processes, and conveys information to and from the components of healthcare system **200.** Database **230** stores the medical information described herein and provides access thereto. The record organizer **232** manages patient medical histories, assists in procedure scheduling, and engages in other operations directed to organizing one or more patient records.

The information system **200** may be implemented as a cloud-based clinical information. A cloud-based clinical information system enables healthcare entities (e.g., patients, clinicians, sites, groups, communities, and/or other entities) to share information via web-based applications, cloud storage and cloud services. For example, a cloud-based clinical information system enables a first clinician to securely upload information into the cloud-based clinical information system to allow a second clinician to view and/or download the information via a web application. Thus, for example, the first clinician may upload an x-ray image into the cloud-based clinical information system, and the second clinician may view the x-ray image via a web browser and/or download the x-ray image onto a local information system employed by the second clinician.

In certain examples, users (e.g., a patient and/or care provider) can access functionality provided by system **200** via a software-as-a-service (SaaS) implementation over a cloud or other computer network, for example. In certain examples, all or part of system **200** can also be provided via platform as a service (PaaS), infrastructure as a service (IaaS), etc. For example, system **200** can be implemented as a cloud-delivered Mobile Computing Integration Platform as a Service. A set of consumer-facing Web-based, mobile, and/or other applications enable users to interact with the PaaS, for example.

While **FIG.** 2 illustrates interconnections between systems and data, the disclosed systems and methods also apply within the context of the Internet Of Things. The Internet Of Things (also referred to as the "Industrial Internet") relates to an interconnection between a device that can use an Internet connection to talk with other devices on the network. Using the connection, devices can communicate to trigger events/actions (e.g., changing temperature, turning on/off, providing a status, etc.). In certain examples, machines can be merged with "big data" to improve efficiency and operations and provide improved data mining.

"Big data" generally refers to a collection of data so large and complex that it becomes difficult to process using traditional data processing tools/methods. Challenges associated with a large data set include data capture, sorting, storage, search, transfer, analysis, and visualization. A trend toward larger data sets is due at least in part to additional information derivable from analysis of a single large set of data, rather than analysis of a plurality of separate, smaller data sets. By analyzing a single large data set, correlations can be found in the data, and data quality can be evaluated.

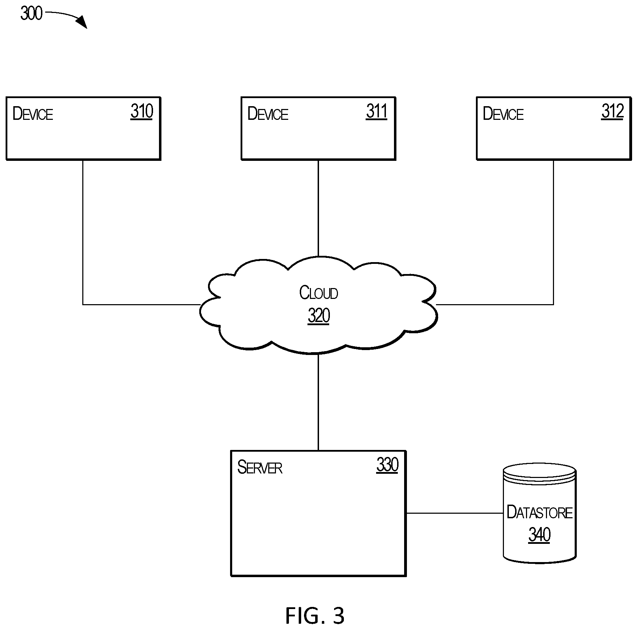

**FIG. 3** is an industrial Internet configuration **300** according to an example embodiment. As with **FIG. 2****,** the industrial Internet configuration **300** is directed to a healthcare-oriented implementation. However, the industrial Internet configuration **300** may be configured to other environments as well, such as a retail store, a factory floor, a construction site, or other such environment. In one embodiment, the configuration **300** includes a plurality of devices **310-312** that access a network cloud **320,** which connects the devices **310-312** with a server **330** and associated data store **340.** In the context of healthcare, devices **310-312** include such devices as pumps, electrocardiographs, inhalers, contact lenses, medicine containers, and other such devices. Other examples of devices **310-312** include printers, RFID tags, RFID readers, scanners, point-of-sale machines, credit card readers, and other such devices. Furthermore, devices **310-312** may also include systems, machines, servers, desktop computers, laptop computers, or combinations of such devices.

In this manner, devices **310-312** become "intelligent" as a network with advanced sensors, controls, analytical-based decision support and hosting software applications. Using such an infrastructure, advanced analytics can be provided to associated data. The analytics combines physics-based analytics, predictive algorithms, automation, and deep domain expertise. Via network cloud **320,** devices **310-312** and associated people can be connected to support more intelligent design, operations, maintenance, and higher server quality and safety, for example.

Using the industrial Internet configuration **300,** for example, a proprietary machine data stream can be extracted from a device **310.** Machine-based algorithms and data analysis are applied to the extracted data. Data visualization of the extracted data can be remote, centralized, etc. The extracted data is then shared with authorized users, and any gathered and/or gleaned intelligence can be provided back to the devices **310-312.**

Imaging informatics includes determining how to tag and index a large amount of data acquired in diagnostic imaging in a logical, structured, and machine-readable format. By structuring data logically, information can be discovered and utilized by algorithms that represent clinical pathways and decision support systems. Data mining can be used to help ensure patient safety, reduce disparity in treatment, provide clinical decision support, etc. Mining both structured and unstructured data from radiology reports, as well as actual image pixel data, can be used to tag and index both imaging reports and the associated images themselves.

The foregoing systems and methods may be applied in the context of a clinical workflow. Clinical workflows are typically defined to include one or more steps or actions to be taken in response to one or more events and/or according to a schedule. Events may include receiving a healthcare message associated with one or more aspects of a clinical record, opening a record(s) for new patient(s), receiving a transferred patient, reviewing and reporting on an image, and/or any other instance and/or situation that requires or dictates responsive action or processing. The actions or steps of a clinical workflow may include placing an order for one or more clinical tests, scheduling a procedure, requesting certain information to supplement a received healthcare record, retrieving additional information associated with a patient, providing instructions to a patient and/or a healthcare practitioner associated with the treatment of the patient, radiology image reading, and/or any other action useful in processing healthcare information.

The defined clinical workflows may include manual actions or steps to be taken by, for example, an administrator or practitioner, electronic actions or steps to be taken by a system or device, and/or a combination of manual and electronic action(s) or step(s). While one entity of a healthcare enterprise may define a clinical workflow for a certain event in a first manner, a second entity of the healthcare enterprise may define a clinical workflow of that event in a second, different manner. In other words, different healthcare entities may treat or respond to the same event or circumstance in different fashions. Differences in workflow approaches may arise from varying preferences, capabilities, requirements or obligations, standards, protocols, etc. among the different healthcare entities.

In various embodiments, a medical exam conducted on a patient can involve review by a healthcare practitioner, such as a radiologist, to obtain, for example, diagnostic information from the exam. In a hospital setting, medical exams can be ordered for a plurality of patients, all of which may be reviewed by an examining practitioner. Each exam has associated attributes, such as a modality, a part of the human body under exam, and/or an exam priority level related to a patient criticality level. Hospital administrators, in managing distribution of exams for review by practitioners, can consider the exam attributes as well as staff availability, staff credentials, and/or institutional factors such as service level agreements and/or overhead costs.

Additional workflows can be facilitated such as bill processing, revenue cycle mgmt., population health management, patient identity, consent management, etc.

The foregoing systems and methods may be deployed to provide real-time location services. Real-time location services (RTLS) facilitate tracking people and assets in an industrial setting, such as a hospital. In one embodiment, an RTLS system is implemented to capture location and proximity information and interactions information from beacon tags installed throughout a hospital. In this embodiment, beacon tags are installed, for example, in the hallways, rooms, equipment, patient wristbands, and in other such locations. In another embodiment, an RTLS system is implemented to capture such location and proximity information from beacon tags installed in a retail store. In this alternative embodiment, the beacon tags are installed, for example, at a point-of-sale machine, within security tags affixed to one or more items, in various aisles, near particular types of inventory, and in other such locations.

Where the beacon tags are installed in a hospital, a caregiver (e.g., a doctor, a nurse, etc.) attaches a beacon badge to themselves (e.g., via a lanyard, a pocket clip, etc.), and as the caregiver performs their duties, the example RTLS system passively captures the location/proximity information and interactions information from the beacon tags. The location/proximity information and interactions information is processed by the beacon badge and sent, via a wireless infrastructure, to a RTLS server. Application event(s) (e.g., interactions between a caregiver and a patient, room level proximity detection, etc.) are generated based on the received location/proximity information and interaction information. In some examples, a beacon badge may be placed on a bed or other asset for which location/proximity information is to be collected between beacon tags and the asset.

In some examples, a beacon badge may be coupled to an extension that facilitates extended battery life for the beacon badge. For example, a hub module is a module with an external power source (e.g., an AC/DC power connection) that can be coupled to a beacon badge via a module connector on the beacon badge and a badge connection on the hub module. The external power source enables the beacon badge to pull power from the hub module rather than its own battery, thereby increasing the battery life and capabilities of the beacon badge. In some examples, a hub module is placed on stationary (or near stationary) equipment, such as a bed, to detect when a patient interacts with the stationary equipment (e.g., moves from the bed). In some examples, the hub module may include one or more sensors to detect additional events. For example, a hub module may be placed on a soap dispenser and include sensors to detect when the soap dispenser performs a dispense event.

The example RTLS system disclosed herein also includes one or more dock modules located throughout an environment. Example dock modules are utilized to charge one or more beacon badges. For example, when a caregiver goes off-duty, the caregiver may dock (e.g., couple) their beacon badge with the dock module, which includes an external power source (e.g., an AC/DC power connection) to charge the beacon badge.

In some examples, the beacon badge is assigned to a caregiver over a period of time (e.g., six months, one year, etc.). In some examples, the beacon badge is assigned to a caregiver as needed. For example, rather than using the same beacon badge during each hospital shift, the caregiver may be assigned a different beacon badge at the start of their shift. In some such examples, the dock module may include additional components to monitor and/or track beacon badge check-in/check-out. For example, the dock module may log when a beacon badge is removed from and/or coupled to the dock module. In some such examples, the dock module includes an identification component enabling a caregiver to identify themselves to the system via a unique identifier (e.g., a hospital identifier, a user identifier, etc.).

For example, at the start of a caregiver's shift, the caregiver may identify themselves to the dock module via a barcode scanner, an RFID reader, near field communication (NFC) reader, a BLE reader, a touchpad, a touch screen interface, etc. of the identification component. When a caregiver is authenticated, the dock module assigns a beacon badge to the caregiver. In some such examples, the dock module and/or the beacon badge may include LEDs to indicate which beacon badge coupled to the dock module is assigned to the caregiver. Additionally or alternatively, the dock module may include a locking mechanism that unlocks a beacon badge when assigned to the caregiver. In some examples, the dock module monitors the charge level (e.g., power level) of the docked beacon badges and assigns a beacon badge to the caregiver based on the charge level. For example, the dock module can determine the beacon badge with the highest charge level and assign that beacon badge first. When a beacon badge is returned to the dock module (e.g., at the end of a shift, when the beacon badge battery reaches a critical or shutdown level, etc.), the dock module disassociates the beacon badge from the caregiver. The dock module may assign the beacon badge to the same caregiver and/or a different caregiver during subsequent use of the beacon badge. The beacon badge can then be charged while it awaits its next use. In some such examples, a returning of the beacon badge to the dock module for recharging triggers a disassociation or unassignment of the beacon badge to the caregiver.

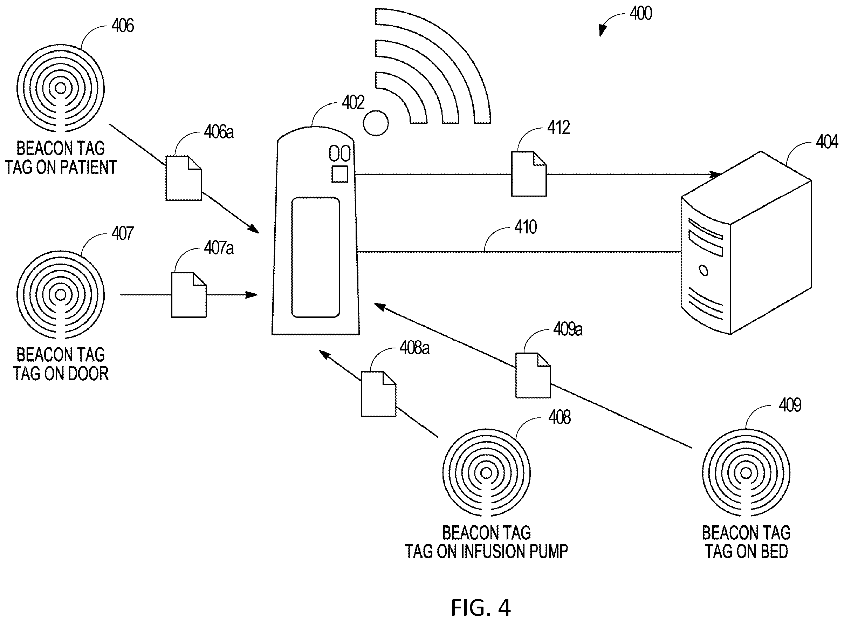

**FIG. 4** is a block diagram illustrating an environment **400** that facilitates proximity detection and location tracking using a mobile wireless bridge, according to an example embodiment. The environment **400** of **FIG. 4** includes a mobile wireless bridge **402** (e.g., a beacon badge), an example real-time locations services (RTLS) server **404** and example beacon tags **406-409.** In the illustrated example of **FIG. 4****,** the RTLS server **404** is a server and/or database that collects and/or receives beacon packets related to proximity/location information and interactions information. In some examples, the RTLS server **404** is implemented using multiple devices. For example, the RTLS server **404** may include disk arrays or multiple workstations (e.g., desktop computers, workstation servers, laptops, etc.) in communication with one another.

In the illustrated example, the beacon badge **402** is in communication with the RTLS server **404** via one or more wireless networks represented by network **410.** Example network **410** may be implemented using any suitable wireless network(s) including, for example, one or more data busses, one or more wireless Local Area Networks (LANs), one or more cellular networks, the Internet, etc. As used herein, the phrase "in communication," including variances thereof (e.g., communicates, in communication with, etc.), encompasses direct communication and/or indirect communication through one or more intermediary components and does not require direct physical (e.g., wired) communication and/or constant communication, but rather additionally includes communication at periodic or aperiodic intervals, as well as one-time events.

In the illustrated example of **FIG. 4****,** the mobile wireless bridge **402** is a beacon badge that receives one or more example beacon messages **406a-409a** from the corresponding source beacon tags **406**-**409**. For example, the beacon tag **406** broadcasts the beacon message **406a,** the beacon tag **407** broadcasts the beacon message **407a,** the beacon tag **408** broadcasts the beacon message **408a** and the beacon tag **409** broadcasts the beacon message **409a.** The example beacon tags **406-409** broadcast the corresponding beacon messages **406a-409a** periodically (e.g., once a second, once a minute, once every ten minutes, etc.). In other examples, the beacon tags **406**-**409** may broadcast the corresponding beacon messages **406a-409a** at different intervals. For example, stationary assets such as a bed or a door may broadcast beacon messages less frequently than mobile assets such as merchandise, medical devices *(e.g.,* IV pumps, an infusion pump, etc.) or other such mobile assets.

In the illustrated example, the beacon messages **406a-409a** include identification information for the source beacon tag **406**-**409**. For example, the beacon message **406a** may include a unique identifier of the beacon tag **406** and location information for the beacon tag **406.** In some examples, the location information may be spatial coordinates (e.g., longitude and latitude coordinates) and/or general location identifiers (e.g., Operating Room #1, patient room **312,** infusion pump #8, men's dressing room, aisle number five, women's dressing room, etc.).

In the illustrated example, the beacon tags **406**-**409** are implemented using low-power BLE transmitters and include a single coin-cell battery. In some examples, the single coin-cell battery provides power to the beacon tags **406**-**409** for two or more years. The beacon tags **406**-**409** are installed throughout an environment. For example, where the environment **400** is a hospital or other healthcare facility, the example beacon tag **406** is located on a patient (e.g., inserted within a patient tag), the example beacon tag **407** is located on a door, the example beacon tag **408** is located on an infusion pump and the example beacon tag **409** is located on a bed. As another example, where the environment **400** is a retail store or other commercial establishment, the example beacon tag **406** is located in the men's changing room, the example beacon tag **407** is located in the women's changing room, the example beacon tag **408** is located near men's clothing, and the example beacon tag **409** is located near the women's clothing.

In the environment **400,** accuracy of the location/proximity information and interaction information can be increased or decreased based on adding or reducing the number of beacon tags placed in the environment.

In the illustrated example, the beacon badge **402** is worn by a hospital caregiver such as a doctor, a nurse, etc. In response to receiving beacon messages (e.g., one or more of the example beacon messages **406a-409a),** the beacon badge 402 generates an example beacon packet **412.** The example beacon packet **412** includes information obtained from the beacon messages **406a-409a,** a timestamp indicating when the respective beacon message was received, and an RSSI value. However, other information may be included in the beacon messages **406a-409a** and/or the beacon packet **412.** Periodically and/or aperiodically, the beacon badge **402** transmits the location/proximity information and interaction information (e.g., the beacon packet **412)** to the RTLS server **404.**

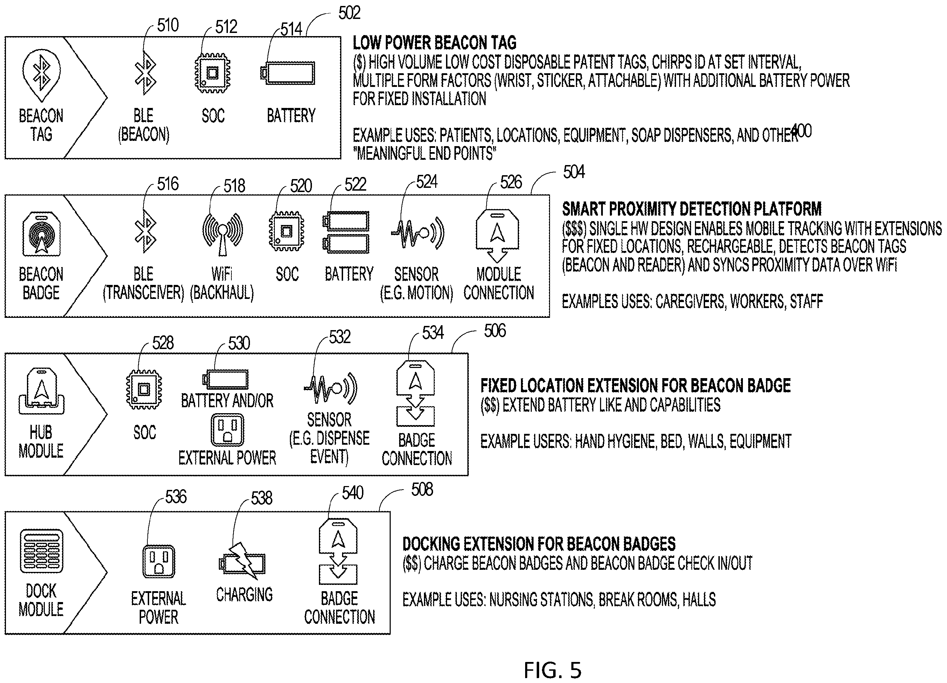

**FIG. 5** illustrates various components included in an example beacon tag **502,** an example beacon badge **504,** an example hub module **506** and example dock module **508.** For example, the beacon tag **502** includes one or more BLE chips (labeled "Beacon") **510** to transmit beacon messages, one or more power sources **514** (e.g., one or more coin-cell batteries) and a system-on-a-chip (SOC) **512** to manage the one or more BLE chips and the one or more power sources. The example beacon badge **504** includes one or more BLE chips **516** (labeled "transceiver") to receive beacon messages, one or more Wi-Fi chips **518** to communicate with a wireless network (e.g., the example network **410 (****FIG. 4****)),** one or more power sources (e.g., one or more batteries) **522,** one or more sensors **524** (e.g., a motion sensor, an accelerometer, a gyroscope, etc.) and a system-on-a-chip (SOC) **524** to manage the one or more BLE chips **516,** the one or more Wi-Fi chips **518,** the one or more power sources **522** and the one or more sensors **524.** The example beacon badge **504** also includes an example module connector **526** to connect the beacon badge **504** to the example hub module **506** and/or the dock module **508.**

In the illustrated example, the beacon badge **504** is connectable to the example hub module **506.** The connection between the beacon badge **504** and the hub module **506** may include a mechanical connection, an electrical connection, or combinations thereof. In the illustrated example, the hub module **506** may be used to track asset interactions with fixed locations. In a healthcare environment, examples of fixed locations include soap dispensers, beds, walls, equipment, etc. In other environments, such as a retail environment, fixed locations may include wall sconces, light fixtures, mirrors, shelving, and other such fixed locations.

The hub module **506** may be leveraged to identify particular locations. As an example, the beacon badge **504** may be coupled, via a badge connection **534,** to a hub module **506** placed on an entrance to a restricted area to identify when a person wearing a beacon tag **502** (or approaches) the restricted area. In one embodiment, the hub module **506** includes a system-on-a-chip (SOC) **528** to manage components of the hub module **506,** one or more power sources **530** (e.g., one or more batteries and an external power source (e.g., an AC/DC connection)) to extend the battery life and capabilities of the beacon badge **504,** one or more sensors **532** communicatively coupled to the SOC **528,** and a badge connection **534** for connecting the beacon badge **504** to the hub module **506.**

In the illustrated example, the beacon badge **504** may be connectable (e.g., mechanically coupled, electronically coupled, etc.) to the example dock module **508.** In the illustrated example, the dock module **508** may be used to charge one or more beacon badges **504.** Accordingly, and in one embodiment, the dock module **508** includes an external power connector **536** *(e.g.,* an AC connector), a charging indicator **538** to indicate whether the beacon badge **504** is charged or charging, and a badge connection **504** for connecting the beacon badge **504** to the dock module **506.** In one embodiment, the dock module **508** is portable. For example, the dock module **506** may be placed throughout one or more environments, such as at cash registers, podiums, counters, nursing stations, break rooms, hallways, etc., and a caregiver may couple their beacon badge **504** to the dock module **508,** via a badge connection, when they are off-duty.

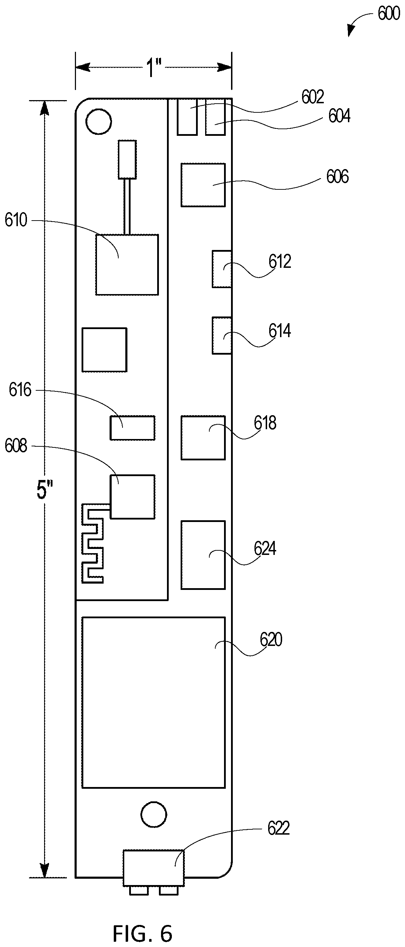

The beacon badge may be deployed as a relatively small and portable electronic device. **FIG. 6** illustrates a beacon badge **600,** according to an example embodiment. In one embodiment, the beacon badge **600** has a dimension of one (1) inch wide by five (5) inches across. However, other dimensions are also possible. The example beacon badge **600** includes example light-emitting diodes (LEDs) **602-604** to indicate status information. For example, the LEDs **602**-**604** may indicate when the battery charge of the beacon badge **600** is low, when the beacon badge **600** is connected to the example RTLS server **404 (****FIG. 4****)** and/or transmitting information (e.g., the example beacon packet **412 (****FIG. 4****))** via the wireless network **410 (****FIG. 4****),** when the beacon badge **600** is receiving and/or processing beacon messages, etc.

The beacon badge **600** includes one or more transceivers for receiving information. In one embodiment, beacon badge **600** receives (e.g., collects, detects, captures, obtains, etc.) beacon messages via an IR sensor **606** and/or a BLE chip **608.** The beacon badge **600** also includes a wireless network transceiver **610** to communicate beacon packets (e.g., the example beacon packet **412)** to the RTLS server **402** via a wireless infrastructure, such as the network **410** of **FIG. 4****.**

The beacon badge **600** also includes user-input controls (e.g., buttons) **612-614** to control the beacon badge **600.** The example beacon badge **600** also includes a motion sensor **616** and an internal measurement unit (IMU) chip **618** (e.g., a gyroscope processor) to manage power utilization of the beacon badge **600.** For example, the motion sensor **616** and/or IMU chip **618** may determine the beacon badge **600** is at rest (e.g., placed on a table) for a period and transition the beacon badge **600** from a normal power utilization state to a low-power utilization state (e.g., a sleep state). The motion sensor **616** and/or IMU chip **618** may transition the beacon badge **600** to a normal power utilization state when the beacon badge **600** is not at rest (e.g., moving with a caregiver). In some examples, transitioning the beacon badge **600** to the low-power utilization state may include deactivating (e.g., turning off) one or more of the transceivers, such as the IR sensor **606,** the BLE chip 608, and/or the wireless transceiver **610,** to conserve power.

The example beacon badge **600** further includes power charging circuitry **620** to manage charging the beacon badge **600** when connected to a power charging device, such as the hub module **506 (****FIG. 5****)** and/or the dock module **508 (****FIG. 5****).** The beacon badge **600** also includes an external connector **622,** such as a micro USB connection, to couple the beacon badge **600** to the hub module **506** and/or the dock module **508.**

The beacon badge **600** also includes a proximity engine **624** to manage received beacon messages (e.g., the example beacon messages **406a-409a (****FIG. 4****)).** In one embodiment, the proximity engine **624** is implemented as firmware residing on machine-readable medium coupled to the beacon badge **600.** Accordingly, the proximity engine **624** may be written in any suitable computer programming and/or computer scripting language. When a beacon message is received, the proximity engine **624** generates a beacon packet **412** to send to the RTLS server **404.** For example, the proximity engine **624** records source beacon tag identification information, such as a unique identifier and location information, from the received beacon message. In some examples, the proximity engine **624** appends a timestamp indicative of when the beacon message was received by the beacon badge **600.** The proximity engine **624** may also determine an RSSI value indicative of the signal strength (e.g., power level) of the received beacon message. In some such examples, the proximity engine **624** also appends the RSSI value to the record for the received beacon message.

In some examples the proximity engine **624** uses the RSSI values to determine proximity of the beacon badge **600** to the source beacon tag. For example, if the RSSI value satisfies a proximity threshold (e.g., the RSSI value is at least a minimum value), the proximity engine **624** determines the source beacon tag is a proximate beacon tag. In some such examples, the proximity engine **624** discards the received beacon message if the RSSI value did not satisfy the proximity threshold. Using the comparison results of the RSSI value and the proximity threshold, the proximity engine **624** filters beacon messages which may be received from source beacon tags located within the environment but to which the beacon badge **600** is not proximate, such as when the beacon badge **600** is worn or carried by a person.

For example, in the context of a healthcare environment, a caregiver may be positioned in one corner of a patient room and the beacon badge **600** may receive a faint beacon message from a source beacon tag positioned at the other corner of the patient room. In some examples, the proximity threshold may vary based on the beacon tag type and the asset on which the beacon tag is placed. For example, the area within which a caregiver is considered to be proximate to a bed may be larger than the area within which a caregiver is considered to be proximate to a telemetry unit. In the illustrated example, the proximity engine **624** collects beacon messages over a period and periodically transmits beacon packets to the RTLS server **402.** Additionally or alternatively, the proximity engine **624** may generate and transmit a beacon packet when a beacon message is received from a proximate source beacon tag.

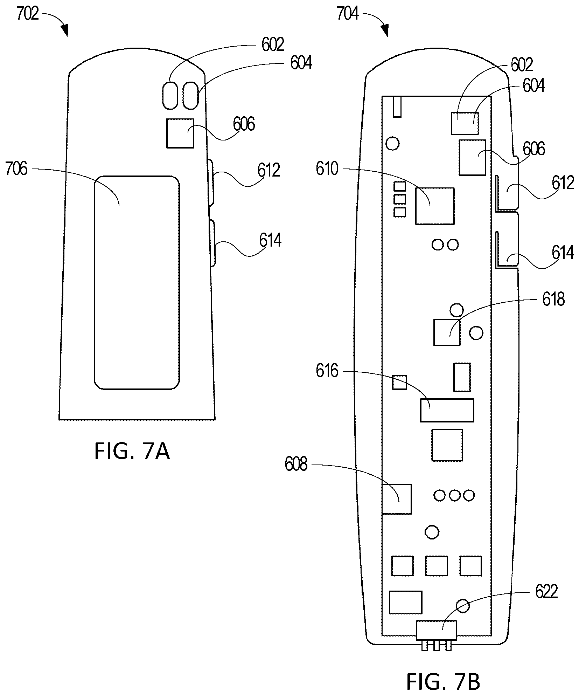

**FIGS**. **7A-7B** illustrate additional details of the beacon badge **600,** according to an example embodiment. In particular, **FIG. 7A** illustrates the outside build **702** of the beacon badge **600** and **FIG. 7B** illustrates a schematic view **704** of the beacon badge **600.** The outside build **702** demonstrates the LEDs **602**-**604**, the IR sensor **606** and the user-input controls **612**-**614** (e.g., depressible buttons). In one embodiment, the outside build **702** also a front clip **706** for securing the beacon badge **600,** such as by securing the beacon badge **600** to an article of clothing, merchandise, a chair, or other such item. For example, the font clip **706** can secure the beacon badge **600** to a doctor's coat pocket.

**FIG. 7B** illustrates example circuitry layout of the components of the beacon badge **600.** In some instances, the example circuitry may differ from the components illustrated in **FIG. 6****;** for example, the schematic view **704** does not include a proximity engine **624.** In some such examples, the beacon badge **600** generates beacon packets and sends them to the RTLS server **404** without filtering (e.g., discarding) the received beacon messages. The example RTLS server **404** may then filter the received beacon messages via a proximity engine available to the RTLS server **404.**

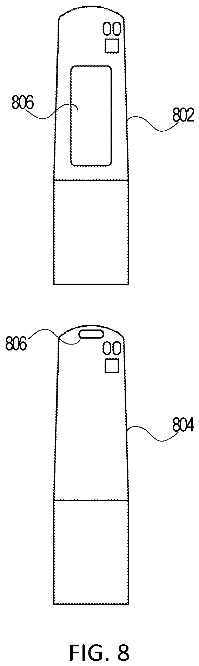

FIG. 8 illustrates alternative implementations **802**-**804** to the beacon badge of **FIG. 6****,** according to further embodiments. In one implementation **802,** the beacon badge includes a front clip **806.** In another implementation **804,** the beacon badge includes lanyard receiver **806.** One example of a lanyard receiver **806** is a hole formed within the surface of the beacon badge. As with the beacon badges illustrated in **FIG. 6** and **FIGS**. **7A****-7B,** the implementations **802**-**804** shown in **FIG. 8** also include LEDs, an IR sensor and user-input controls.

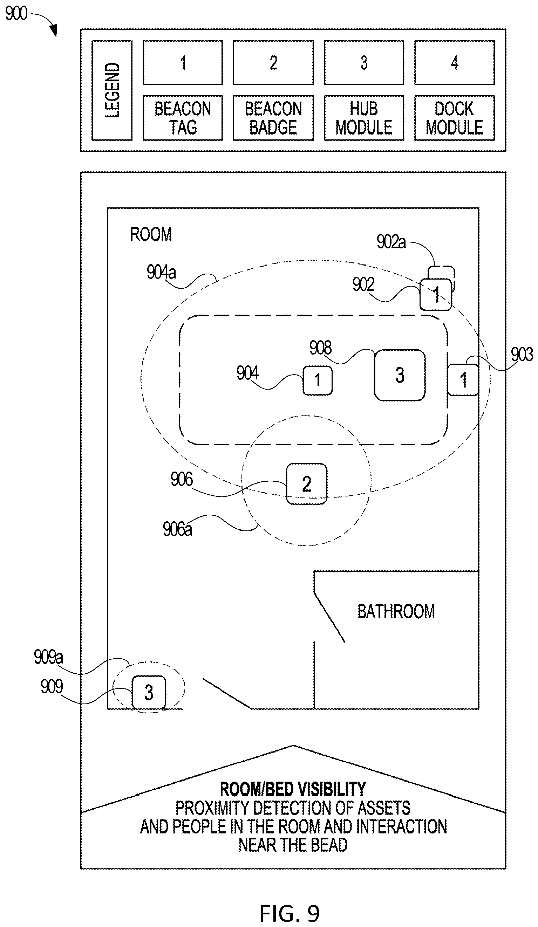

The beacon badge illustrated in the foregoing figures may be used in a variety of environments. Accordingly, **FIG. 9** illustrates an example environment **900** to facilitate proximity detection and location tracking using a mobile wireless bridge, according to an example embodiment. In one embodiment, the environment **900** is a healthcare facility. Thus, as shown in **FIG. 9****,** the environment **900** includes a beacon tag **902** placed on equipment (e.g., an infusion pump, a telemetry unit, etc.), a beacon tag **903** placed at a location (e.g., against a wall) and a beacon tag **904** placed on a patient (e.g., via a patient tag). The environment **900** also includes a beacon badge **906** placed on a caregiver and hub modules **908, 909** placed on a bed and on a hand hygiene dispenser, respectively. In the illustrated example, each of the beacon tags **902**-**904**, the beacon badge **906** and the hub modules **908, 909** are associated with a proximity area. The proximity area for a beacon tag corresponds to the area around the beacon tag in which the beacon tag broadcasts its beacon messages and in which a received beacon message has an RSSI value that satisfies a proximity threshold. The proximity area for a beacon badge corresponds to the area around the beacon badge in which the caregiver may be considered to interacting with an asset. The proximity area for a hub module corresponds to the area around the hub module in which an asset is considered to be proximate to the hub module.

As shown in **FIG. 9****,** the size of the proximity areas for the beacon tags **902-904,** the beacon badge **906,** and the hub modules **908**-**909** are different. For example, the proximity area **909a,** corresponding to the area within which an asset is considered to be proximate to the hand hygiene dispenser, is less than the proximity area **904a,** corresponding to the area within which a caregiver is considered to be interacting with an asset (e.g., a patient).

In **FIG. 9****,** a caregiver is considered to be interacting with an asset when the proximity area (e.g., proximity area **906a)** generated by their beacon badge overlaps with the proximity area generated by the source beacon tag placed on the asset. For example, the proximity area **906a** (e.g., corresponding to the caregiver) is overlapping with the proximity area **904a** (e.g., corresponding to the patient), and, thus, the caregiver is determined to be proximate to the patient and interacting with the patient. In this example, because the proximity area **906a** (e.g., corresponding to the caregiver) does not overlap with example proximity area **902** (e.g., generated by the beacon tag **902),** the caregiver is considered not be interacting with the equipment on which the beacon tag **902** is placed. Furthermore, because of the interactions information, it can also be determined where the caregiver was positioned while interacting with the patient.

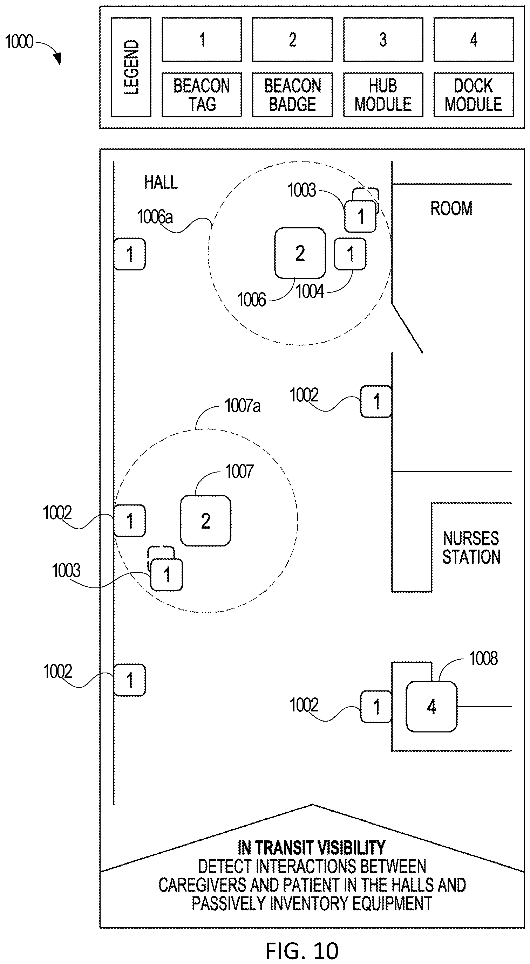

**FIG. 10** illustrates another example environment **1000** to facilitate proximity detection and location tracking using a mobile wireless bridge, according to another example embodiment. In particular, and in the context of providing healthcare, **FIG. 10** illustrates an example transit environment **1000** (e.g., a hallway) in which one or more caregivers may be interacting with assets. The transit environment **1000** includes beacon tags **1002** positioned at different locations in the transit environment **1000,** a beacon tag **1003** positioned on equipment, and a beacon tag **1004** positioned on a patient.

Various beacon badges **1006, 1007** are provided to caregivers to wear throughout their normal workflow. The transit environment **1000** also includes a dock module **1008** to which one or more of the beacon badges **1006, 1007** are coupled *(e.g.,* for charging).

As a caregiver walks through the transit environment **1000,** the interactions between the beacon tags **1002-1003** and beacon badges **1006,1007** are used to determine that the caregiver is proximate to and interacting with different assets based on, for example, overlapping proximity areas. For example, overlap between the proximity area **1006a** and the proximity area generated by the beacon tag **1003** can be used to determine when the caregiver is proximate to equipment on which the beacon tag **1003** resides (e.g., a telemetry unit, a wheelchair, etc.). As another example, overlap between the proximity area **1006a** and the proximity area generated by the beacon tag **1004** can be used to determine when the caregiver is proximate to and/or interacting with a patient. As a further example, overlap between example proximity area **1007a** (e.g., generated by the beacon badge **1007)** and the proximity area generated by the beacon tag **1002** can be used to determine when the caregiver is proximate to a location (e.g., a wall, a doorway, etc.), etc. In this manner, the interactions between the beacon badges and the beacon tags indicate where a given caregiver is relative to other items in the environment **1000.**

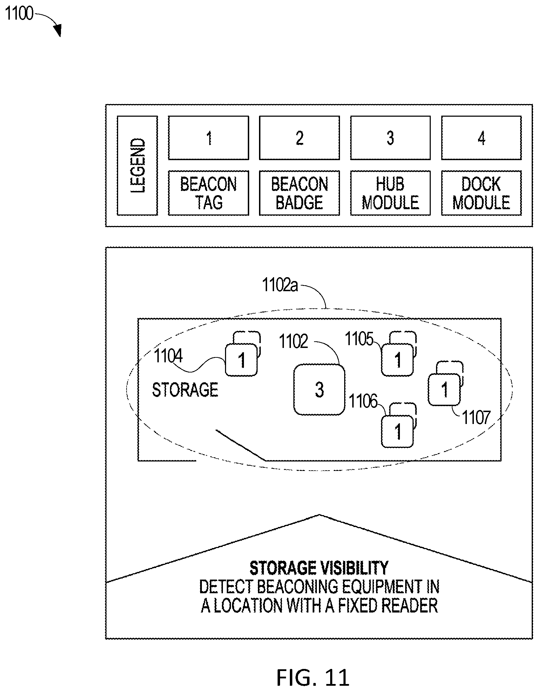

**FIG. 11** illustrates yet a further environment **1100** to facilitate proximity detection and location tracking using a mobile wireless bridge, according to a further embodiment. In particular, **FIG. 11** illustrates a storage environment **1100** that includes a hub module **1102,** having a proximity area **1102a,** and beacon tags **1104** placed on different equipment. In the context of healthcare, such equipment might include IV pumps, wheelchairs, telemetry units, etc. In the context of other environments, such as retail, such equipment might include cash registers, office supplies, chairs, tables, signage, and other such equipment.

In **FIG. 11****,** a beacon badge (not shown) may be coupled to the hub module **1102,** thereby causing the hub module **1102** to detect proximate assets (e.g., the respective equipment on which the beacon tags **1104-1107** are placed). Using the coupling of the beacon badge to the hub module **1102,** the interactions between the hub module **1102** and the beacon tags **1104-1107** can be used to determine when equipment is removed from the storage environment **1100** (e.g., the proximity area of the beacon tag affixed to the equipment does not overlap with the proximity area **1102a** of the hub module **1102).** Through these interactions, it can also be determined when an asset is moved into the storage environment **1100** (e.g., the proximity area of the beacon tag affixed to the equipment being placed into storage overlaps with the proximity area **1102a).**

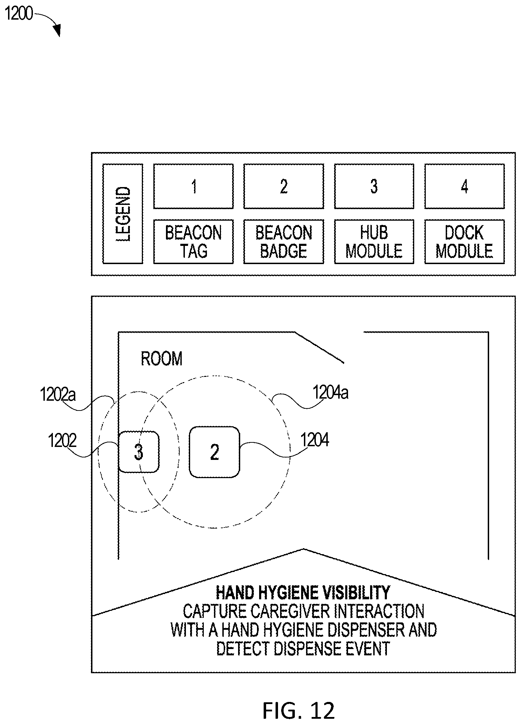

**FIG.** 12 illustrates another environment **1200** to facilitate proximity detection and location tracking using a mobile wireless bridge, according to another embodiment. In particular, and in the context of healthcare, **FIG. 12** illustrates a hand hygiene dispensing environment **1200.** In the environment **1200,** a hub module **1202** is positioned on (or near) a hand hygiene dispenser (e.g., a soap dispenser) and generates a proximity area **1202a.** The environment **1200** also includes a caregiver wearing a beacon badge **1204,** which generates a proximity area **1204a.** In **FIG. 12****,** when the proximity area **1202a** and the proximity area **1204a** overlap, an interaction is detected. For example, the beacon badge **1204** receives a beacon message from the beacon tag coupled to the hub module **1202,** and determines an RSSI value corresponding to the received beacon message. The reception of the beacon message may be considered an interaction with the beacon tag coupled to the hub module **1202.** In addition, a dispense event may also be detected based on, for example, additional sensors included in the hub module **1202** and/or based on a predetermined time threshold in which the beacon badge **1204** is in proximity to the hub module **1202.**

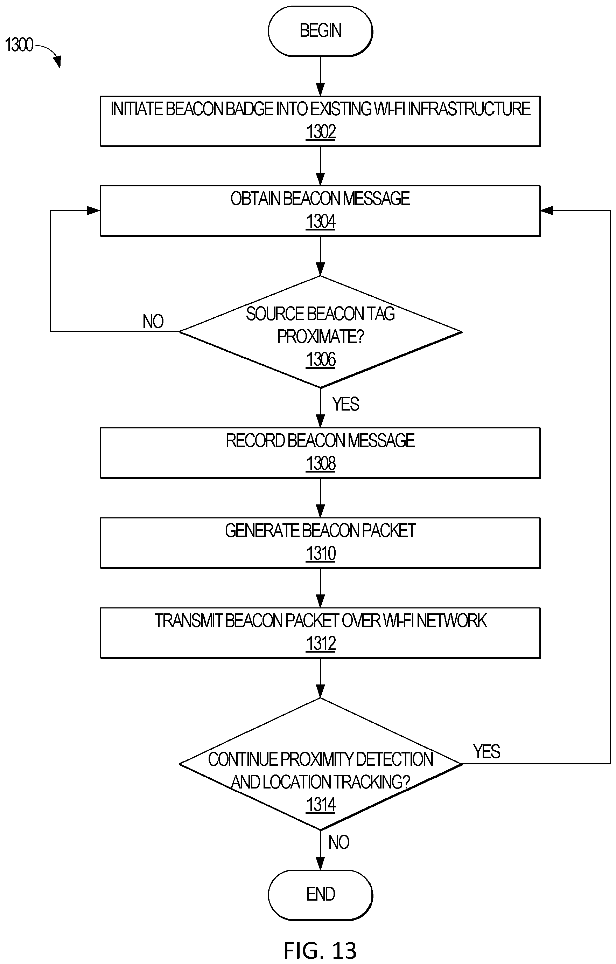

**FIG. 13** illustrates a method **1300** for operating the disclosed wireless bridge according to an example embodiment. The method **1302** is implemented by one or more of a beacon tag, a beacon module, a hub module, and/or a dock module, and is discussed by way of reference thereto. Method **1300** begins at block **1302** when the beacon badge **402 (****FIG. 4****)** is initiated. For example, the beacon badge **402** may be configured to communicate with the example RTLS server **404 (****FIG. 4****)** via the network **410 (****FIG. 4****).** At block **1304,** the beacon badge **402** obtains a beacon message (e.g., the example beacon messages **406a-409a (****FIG. 4****)).** For example, the beacon badge **402** may include an IR sensor and/or a BLE chip to receive beacon messages broadcast using BLE technology. At block **1306,** the beacon badge **402** determines whether the source beacon tag corresponding to the obtained beacon message is proximate. For example, the beacon badge **402** may include the proximity engine **602 (****FIG. 6****),** and determine an RSSI value for the received beacon message. If, at block **1306,** the proximity engine **602** determines the source beacon tag is not proximate to the beacon badge (e.g., the RSSI value did not satisfy a proximity threshold), then, the beacon message is discarded and control returns to block **1304** to wait to obtain another beacon message.

If, at block **1306,** the proximity engine **602** determines that the source beacon tag is proximate to the beacon badge (e.g., the RSSI value satisfied the proximity threshold), then, at block **1308,** the proximity engine **602** records the beacon message. For example, the proximity engine **602** may log the unique identifier and location information included in the obtained beacon message. The example proximity engine **602** may also append a timestamp and an RSSI value to the record. At block **1310,** the proximity engine **602** generates the example beacon packet **412 (****FIG. 4****).** For example, the proximity engine **602** aggregates the records for beacon messages obtained over a period. In some examples, the proximity engine **602** generates the beacon packet **412** in response to receiving the beacon message. At block **1312,** the proximity engine **602** transmits the beacon packet **412** to the RTLS server **404 (****FIG. 4****)** over the Wi-Fi network **410.** At block **1314,** the proximity engine **612** determines whether to continue proximity detection and location tracking in the environment. For example, the motion sensor and/or IMU chip may determine that the beacon badge **402** is at rest for a threshold period. If, at block **1314,** the proximity engine **612** determined to continue proximity detection and location tracking, then control returns to block **1304** to wait to obtain another beacon message. If, at block **1314,** the proximity engine **612** determined to stop proximity detection and location tracking, then the method **1300** ends.

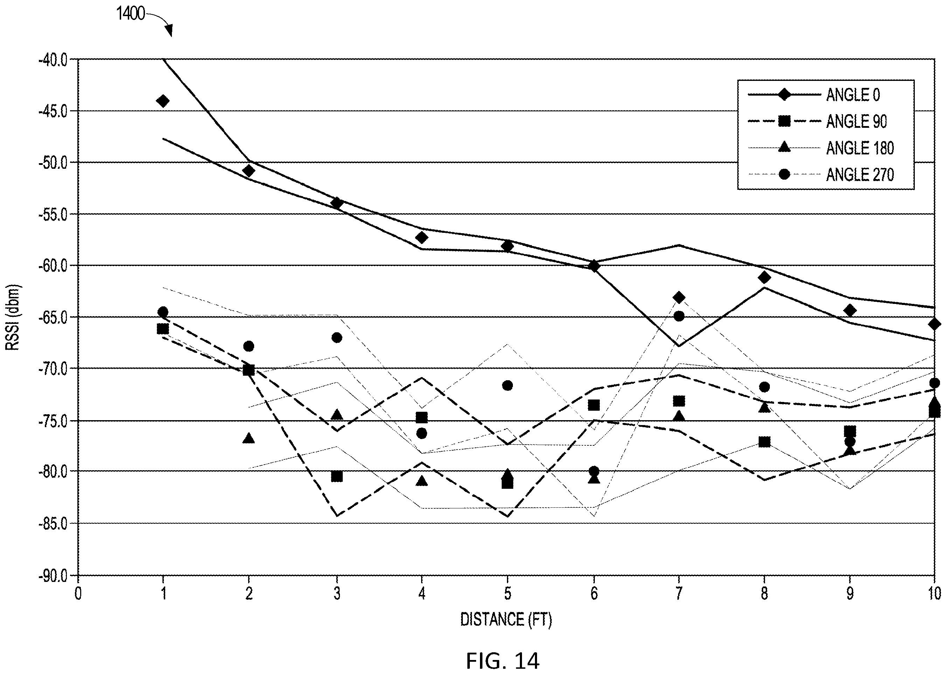

**FIG. 14** is graph **1400** correlating average RSSI values with distance based on the angle at a beacon badge receives a signal from a beacon tag. In **FIG. 14****,** the graph **1400** also displays the 95% upper and lower bounds of the RSSI values measured at different distances.

In operation, a real-time location service (RTLS) system can be used for proximity detection and location tracking in various types of environments, such as retail stores, factory floors, hospitals, industrial centers, and other such environments. The RTLS system disclosed herein may be used to track the location of assets (e.g., equipment, tools, merchandise, inventory, supplies, personnel, patients, employees, etc.) within the environment. To track the location of the assets, the RTLS system includes beacon tags placed (e.g., positioned on, affixed to, coupled with, etc.) on the respective assets. The beacon tags periodically broadcast a beacon message including identification information for the corresponding source beacon tag. For example, a beacon message may include a unique identifier and location information for the beacon tag that broadcast the beacon message.

The disclosed RTLS system also includes one or more beacon badges, which may be worn by personnel moving through or working in the environment. The beacon badges include an IR sensor to receive the broadcast beacon messages. As the personnel move about the environment, their respective beacon badges obtain beacon messages. In certain embodiments, the beacon badge includes a proximity engine to manage the obtained beacon messages. For example, the proximity engine may determine an RSSI value for an obtained message and compare the RSSI value to a proximity threshold. If the RSSI value does not satisfy the proximity threshold, the beacon message may be discarded by the proximity engine. In other examples, if the RSSI value does satisfy the proximity threshold, the proximity engine records information included in the beacon message such as the unique identifier and the location information. In certain embodiments, the proximity engine may also log a timestamp indicative of when the beacon message was received and the RSSI value for the beacon message. Periodically and/or aperiodically, the proximity engine generates a beacon packet from the recorded beacon messages.

In certain embodiments, the proximity engine transmits the beacon packets over a wireless network to an RTLS server. Using the environment's wireless network to transmit the beacon packets helps manage costs for the RTLS system, as a new network infrastructure does not need to be built or implemented. The RTLS server aggregates the beacon packets received from one or more beacon badges in the environment and generates reports based on the received beacon packets. For example, an asset (e.g., a wheelchair, a jackhammer, an article of clothing, etc.) may be "tagged" with a beacon tag having a unique identifier. When the location of the asset needs to be determined, the RTLS server can generate a report identifying the beacon messages with the matching unique identifier and identify the location of the asset. For example, the RTLS server can determine whether the asset is being moved, the last time a given personnel was near the asset, or where the asset is likely to be located.

In some examples, the RTLS systems may detect proximity and interaction with assets in the environment. In one embodiment, hub modules enable coupling a beacon badge to an extension having an external power source. For example, a hub module may be positioned on a doorway, a shelf, a ledge, in a room, or elsewhere within the environment. The hub module also includes an AC/DC connection to draw power. By coupling the beacon badge to the hub module, the battery life of the beacon badge can be extended. In certain embodiments, the beacon badge is coupled to the hub module so that the hub module receives beacon messages as assets move near the hub. For example, an employee may be provided with an identification tag that includes a beacon tag. When the employee moves near a doorway or other asset, the hub module receives beacon messages broadcast by the beacon tag. Using the received beacon messages, it can be determined where the employee is going.

In certain embodiments, the proximity engine included in the beacon badge may determine whether a source beacon tag is proximate to the beacon badge. For example, when the proximity area of the beacon tag overlaps with the proximity area of the beacon badge, a determination can be made that an employee wearing the beacon badge is proximate and/or interacting with the asset on which the beacon tag is placed.

Although examples disclosed herein include the proximity engine in the beacon badge, the proximity engine may additionally or alternatively be included with the RTLS server. For example, if the beacon badge does not include a proximity engine, the beacon badge can transmit beacon packets as a beacon message is received. In some such examples, the RTLS server may process the beacon packets and determine whether to discard the corresponding beacon message information and/or detect proximity of beacon tags with beacon badges. In some examples, the beacon badge may include proximity engine to filter out beacon messages received with weak RSSI values, and the RTLS server may include a proximity engine to determine when a beacon badge and a beacon tag are proximate.

### MODULES, COMPONENTS, AND LOGIC

Certain embodiments are described herein as including logic or a number of components, modules, or mechanisms. Modules may constitute either software modules (e.g., code embodied on a machine-readable medium) or hardware modules. A "hardware module" is a tangible unit capable of performing certain operations and may be configured or arranged in a certain physical manner. In various example embodiments, one or more computer systems (e.g., a standalone computer system, a client computer system, or a server computer system) or one or more hardware modules of a computer system (e.g., a processor or a group of processors) may be configured by software (e.g., an application or application portion) as a hardware module that operates to perform certain operations as described herein.

In some embodiments, a hardware module may be implemented mechanically, electronically, or any suitable combination thereof. For example, a hardware module may include dedicated circuitry or logic that is permanently configured to perform certain operations. For example, a hardware module may be a special-purpose processor, such as a Field-Programmable Gate Array (FPGA) or an Application Specific Integrated Circuit (ASIC). A hardware module may also include programmable logic or circuitry that is temporarily configured by software to perform certain operations. For example, a hardware module may include software executed by a general-purpose processor or other programmable processor. Once configured by such software, hardware modules become specific machines (or specific components of a machine) uniquely tailored to perform the configured functions and are no longer general-purpose processors. It will be appreciated that the decision to implement a hardware module mechanically, in dedicated and permanently configured circuitry, or in temporarily configured circuitry (e.g., configured by software) may be driven by cost and time considerations.

Accordingly, the phrase "hardware module" should be understood to encompass a tangible entity, be that an entity that is physically constructed, permanently configured (e.g., hardwired), or temporarily configured (e.g., programmed) to operate in a certain manner or to perform certain operations described herein. As used herein, "hardware-implemented module" refers to a hardware module. Considering embodiments in which hardware modules are temporarily configured (e.g., programmed), each of the hardware modules need not be configured or instantiated at any one instance in time. For example, where a hardware module comprises a general-purpose processor configured by software to become a special-purpose processor, the general-purpose processor may be configured as respectively different special-purpose processors (e.g., comprising different hardware modules) at different times. Software accordingly configures a particular processor or processors, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple hardware modules exist contemporaneously, communications may be achieved through signal transmission (e.g., over appropriate circuits and buses) between or among two or more of the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions described herein. As used herein, "processor-implemented module" refers to a hardware module implemented using one or more processors.

Similarly, the methods described herein may be at least partially processor-implemented, with a particular processor or processors being an example of hardware. For example, at least some of the operations of a method may be performed by one or more processors or processor-implemented modules. Moreover, the one or more processors may also operate to support performance of the relevant operations in a "cloud computing" environment or as a "software as a service" (SaaS). For example, at least some of the operations may be performed by a group of computers (as examples of machines including processors), with these operations being accessible via a network (e.g., the Internet) and via one or more appropriate interfaces (e.g., an Application Program Interface (API)).

The performance of certain of the operations may be distributed among the processors, not only residing within a single machine, but deployed across a number of machines. In some example embodiments, the processors or processor-implemented modules may be located in a single geographic location (e.g., within a home environment, an office environment, or a server farm). In other example embodiments, the processors or processor-implemented modules may be distributed across a number of geographic locations.

### MACHINE AND SOFTWARE ARCHITECTURE

The modules, methods, applications and so forth described in conjunction with **FIGS. 1-15** are implemented in some embodiments in the context of a machine and an associated software architecture. The sections below describe representative software architecture(s) and machine (e.g., hardware) architecture that are suitable for use with the disclosed embodiments.

Software architectures are used in conjunction with hardware architectures to create devices and machines tailored to particular purposes. For example, a particular hardware architecture coupled with a particular software architecture will create a mobile device, such as a mobile phone, tablet device, or so forth. A slightly different hardware and software architecture may yield a smart device for use in the "internet of things." While yet another combination produces a server computer for use within a cloud computing architecture. Not all combinations of such software and hardware architectures are presented here as those of skill in the art can readily understand how to implement the invention in different contexts from the disclosure contained herein.

### EXAMPLE MACHINE ARCHITECTURE AND MACHINE-READABLE MEDIUM

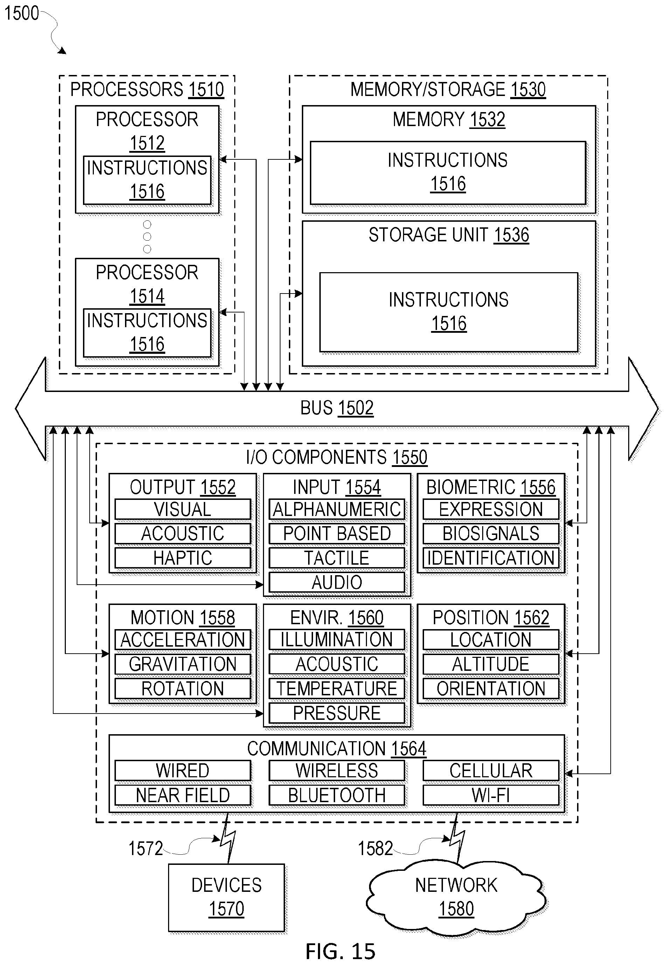

**FIG. 15** is a block diagram illustrating components of a machine **1500,** according to some example embodiments, able to read instructions from a machine-readable medium (e.g., a machine-readable storage medium) and perform any one or more of the methodologies discussed herein. Specifically, **FIG. 15** shows a diagrammatic representation of the machine **1500** in the example form of a computer system, within which instructions **1516** (e.g., software, a program, an application, an applet, an app, or other executable code) for causing the machine **1500** to perform any one or more of the methodologies discussed herein may be executed. For example the instructions may cause the machine to execute the flow diagrams of **FIG. 14****.** Additionally, or alternatively, the instructions may implement one or more of the components shown in **FIGS. 5-6****,** and so forth. The instructions transform the general, non-programmed machine into a particular machine programmed to carry out the described and illustrated functions in the manner described. In alternative embodiments, the machine **1500** operates as a standalone device or may be coupled (e.g., networked) to other machines. In a networked deployment, the machine **1500** may operate in the capacity of a server machine or a client machine in a server-client network environment, or as a peer machine in a peer-to-peer (or distributed) network environment. The machine **1500** may comprise, but not be limited to, a server computer, a client computer, a personal computer (PC), a tablet computer, a laptop computer, a personal digital assistant (PDA), a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions **1516,** sequentially or otherwise, that specify actions to be taken by machine **1500.** Further, while only a single machine **1500** is illustrated, the term "machine" shall also be taken to include a collection of machines **1500** that individually or jointly execute the instructions **1516** to perform any one or more of the methodologies discussed herein.

The machine **1500** may include processors **1510,** memory **1530,** and I/O components **1550,** which may be configured to communicate with each other such as via a bus **1502.** In an example embodiment, the processors **1510** (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an Application Specific Integrated Circuit (ASIC), a Radio-Frequency Integrated Circuit (RFIC), another processor, or any suitable combination thereof) may include, for example, processor **1512** and processor **1514** that may execute instructions **1516.** The term "processor" is intended to include multi-core processor that may comprise two or more independent processors (sometimes referred to as "cores") that may execute instructions contemporaneously. Although **FIG. 15** shows multiple processors, the machine **1500** may include a single processor with a single core, a single processor with multiple cores (e.g., a multi-core process), multiple processors with a single core, multiple processors with multiples cores, or any combination thereof.

The memory/storage **1530** may include a memory **1532,** such as a main memory, or other memory storage, and a storage unit **1536,** both accessible to the processors **1510** such as via the bus **1502.** The storage unit **1536** and memory **1532** store the instructions **1516** embodying any one or more of the methodologies or functions described herein. The instructions **1516** may also reside, completely or partially, within the memory **1532,** within the storage unit **1536,** within at least one of the processors **1510** (e.g., within the processor's cache memory), or any suitable combination thereof, during execution thereof by the machine **1500.** Accordingly, the memory **1532,** the storage unit **1536,** and the memory of processors **1510** are examples of machine-readable media.

As used herein, "machine-readable medium" means a device able to store instructions and data temporarily or permanently and may include, but is not be limited to, random-access memory (RAM), read-only memory (ROM), buffer memory, flash memory, optical media, magnetic media, cache memory, other types of storage (e.g., Erasable Programmable Read-Only Memory (EEPROM)) and/or any suitable combination thereof. The term "machine-readable medium" should be taken to include a single medium or multiple media (e.g., a centralized or distributed database, or associated caches and servers) able to store instructions **1516.** The term "machine-readable medium" shall also be taken to include any medium, or combination of multiple media, that is capable of storing instructions (e.g., instructions **1516)** for execution by a machine (e.g., machine **1500),** such that the instructions, when executed by one or more processors of the machine **1500** (e.g., processors **1510),** cause the machine **1500** to perform any one or more of the methodologies described herein. Accordingly, a "machine-readable medium" refers to a single storage apparatus or device, as well as "cloud-based" storage systems or storage networks that include multiple storage apparatus or devices. The term "machine-readable medium" excludes signals per se.

The I/O components **1550** may include a wide variety of components to receive input, provide output, produce output, transmit information, exchange information, capture measurements, and so on. The specific I/O components **1550** that are included in a particular machine will depend on the type of machine. For example, portable machines such as mobile phones will likely include a touch input device or other such input mechanisms, while a headless server machine will likely not include such a touch input device. It will be appreciated that the I/O components **1550** may include many other components that are not shown in **FIG. 15****.** The I/O components **1550** are grouped according to functionality merely for simplifying the following discussion and the grouping is in no way limiting. In various example embodiments, the I/O components **1550** may include output components **1552** and input components **1554.** The output components **1552** may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components **1554** may include alphanumeric input components (e.g., a keyboard, a touch screen configured to receive alphanumeric input, a photo-optical keyboard, or other alphanumeric input components), point based input components (e.g., a mouse, a touchpad, a trackball, a joystick, a motion sensor, or other pointing instrument), tactile input components (e.g., a physical button, a touch screen that provides location and/or force of touches or touch gestures, or other tactile input components), audio input components (e.g., a microphone), and the like.

In further example embodiments, the I/O components **1550** may include biometric components **1556,** motion components **1558,** environmental components **1560,** or position components **1562** among a wide array of other components. For example, the biometric components **1556** may include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram based identification), and thelike. The motion components **1558** may include acceleration sensor components (e.g., accelerometer), gravitation sensor components, rotation sensor components (e.g., gyroscope), and so forth. The environmental components **1560** may include, for example, illumination sensor components (e.g., photometer), temperature sensor components (e.g., one or more thermometer that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components **1562** may include location sensor components (e.g., a Global Position System (GPS) receiver component), altitude sensor components (e.g., altimeters or barometers that detect air pressure from which altitude may be derived), orientation sensor components (e.g., magnetometers), and the like.

Communication may be implemented using a wide variety of technologies. The I/O components **1550** may include communication components **1564** operable to couple the machine **1500** to a network **1580** or devices **1570** via coupling **1582** and coupling **1572** respectively. For example, the communication components **1564** may include a network interface component or other suitable device to interface with the network **1580.** In further examples, communication components **1564** may include wired communication components, wireless communication components, cellular communication components, Near Field Communication (NFC) components, Bluetooth® components (e.g., Bluetooth® Low Energy), Wi-Fi® components, and other communication components to provide communication via other modalities. The devices **1570** may be another machine or any of a wide variety of peripheral devices (e.g., a peripheral device coupled via a Universal Serial Bus (USB)).

Moreover, the communication components **1564** may detect identifiers or include components operable to detect identifiers. For example, the communication components **1564** may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, **PDF417,** Ultra Code, UCC RSS-2D bar code, and other optical codes), or acoustic detection components (e.g., microphones to identify tagged audio signals). In addition, a variety of information may be derived via the communication components **1564,** such as, location via Internet Protocol (IP) geo-location, location via Wi-Fi® signal triangulation, location via detecting a NFC beacon signal that may indicate a particular location, and so forth.

### TRANSMISSION MEDIUM

In various example embodiments, one or more portions of the network **1580** may be an ad hoc network, an intranet, an extranet, a virtual private network (VPN), a local area network (LAN), a wireless LAN (WLAN), a wide area network (WAN), a wireless WAN (WWAN), a metropolitan area network (MAN), the Internet, a portion of the Internet, a portion of the Public Switched Telephone Network (PSTN), a plain old telephone service (POTS) network, a cellular telephone network, a wireless network, a Wi-Fi® network, another type of network, or a combination of two or more such networks. For example, the network **1580** or a portion of the network **1580** may include a wireless or cellular network and the coupling **1582** may be a Code Division Multiple Access (CDMA) connection, a Global System for Mobile communications (GSM) connection, or other type of cellular or wireless coupling. In this example, the coupling **1582** may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard setting organizations, other long range protocols, or other data transfer technology.

The instructions **1516** may be transmitted or received over the network **1580** using a transmission medium via a network interface device (e.g., a network interface component included in the communication components **1564)** and utilizing any one of a number of well-known transfer protocols (e.g., hypertext transfer protocol (HTTP)). Similarly, the instructions **1516** may be transmitted or received using a transmission medium via the coupling **1572** (e.g., a peer-to-peer coupling) to devices **1570.** The term "transmission medium" shall be taken to include any intangible medium that is capable of storing, encoding, or carrying instructions **1516** for execution by the machine **1500,** and includes digital or analog communications signals or other intangible medium to facilitate communication of such software.

### LANGUAGE

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components.

## Claims

1. An electronic device (402, 600, 702, 704) comprising:
a wireless receiver (606, 608), the wireless receiver configured to use a first wireless protocol and receive a first wireless message (406a, 407a, 408a, 409a), the first wireless message including an identifier that identifies a device (406, 407, 408, 409) having sent the first wireless message;
a wireless transmitter (610), the wireless transmitter configured to use a second wireless protocol and send a second wireless message (412) to a real time location services, RTLS, server, the second wireless message including the identifier, where the first wireless protocol and the second wireless protocol are different wireless protocols; and
at least one hardware processor (618, 624)
configured to determine a received signal strength indication indicating a signal strength of a wireless signal used to send the first wireless message
**characterized in that** the hardware processor (618, 624) is further configured to
activate the wireless transmitter based on the wireless receiver having received the first wireless message and based on a comparison of the received signal strength indication with a signal strength threshold.

2. The electronic device (402, 600, 702, 704) of claim 1, wherein the first wireless protocol includes Bluetooth and the second wireless protocol includes at least one wireless protocol selected from the group of Institute of Electrical and Electronics Engineers, IEEE, 802.11 protocols.

3. The electronic device (402, 600, 702, 704) of claim 1, further comprising: a motion sensor (616) configured to detect movement, wherein the at least one hardware processor (618, 624) transitions at least one of the wireless receiver or the wireless transmitter from a first state to a second state based on whether the motion sensor detects movement.

4. The electronic device (402, 600, 702, 704) of claim 3, wherein the first state comprises a deactivation state in which at least one of the wireless receiver or the wireless transmitter operates at a reduced power and the second state comprises an activation state in which at least one of the wireless receiver (606, 608) or the wireless transmitter (610) operates at operational power.

5. The electronic device (402, 600, 702, 704) of claim 1, further comprising at least two illuminated indicators (602, 604) activatable by the at least one hardware processor, the at least two illuminated indicators (538) indicating a status of at least one of the wireless receiver or the wireless transmitter.

6. The electronic device (402, 600, 702, 704) of claim 1, wherein:
the at least one processor (618, 624) is electrically connected to the wireless transmitter (610) and the wireless receiver (606, 608);
the at least one hardware processor, the wireless transmitter, and the wireless receiver are housed within an enclosed casing having at least two surfaces; and the casing includes at least one flexible clip (706) affixed to at least one of the at least two surfaces, wherein the enclosed casing is securable to an article of clothing using the at least one flexible clip.

7. A real-time location services, RTLS, system (400) comprising:
a plurality of beacon tags (406, 407, 408, 409), configured to broadcast at least one wireless message (406a, 407a, 408a, 409a), the at least one wireless message being broadcast using a first wireless protocol; and
an electronic device (402, 600, 702, 704) according to any of claims 1 to 6.

8. The system (400) of claim 7, wherein at least one of the plurality of beacon tags (406, 407, 408, 409) is configured to periodically broadcast the at least one wireless message (406a, 407a, 408a, 409a) at a fixed time interval.

9. A method (1300) performed by an electronic device (402, 600, 702, 704), comprising:
receiving (1304) a first wireless message (406a, 407a, 408a, 409a) by a wireless receiver (606, 608) of the electronic device (402, 600, 702, 704) using a first wireless protocol, the first wireless message including an identifier that identifies a device (406, 407, 408, 409) having sent the first wireless message;
determining a received signal strength indication indicating a signal strength of a wireless signal used to send the first wireless message;
transmitting (1312) a second wireless message (412) by a wireless transmitter (610) of the electronic device (402, 600, 702, 704) to a real time location services, RTLS, server using a second wireless protocol, the second wireless message including the identifier, where the first wireless protocol and the second wireless protocol are different wireless protocols;
**characterized in that** the method further comprises: activating, by at least one hardware processor (618, 624) of the electronic device (402, 600, 702, 704), the wireless transmitter based on the wireless receiver having received the first wireless message and based on a comparison (1306) of the received signal strength indication with a signal strength threshold.

10. The method (1300) of claim 9, wherein the first wireless protocol includes Bluetooth and the second wireless protocol includes at least one wireless protocol selected from the group of Institute of Electrical and Electronics Engineers, IEEE, 802.11 protocols.

11. The method (1300) of claim 9, further comprising:
detecting movement by a motion sensor (616) of the electronic device (402 600, 702, 704); and
transitioning, by the at least one hardware processor (618, 624), at least one of the wireless receiver (606, 608) or the wireless transmitter (610) from a first state to a second state based on the movement detected by the motion sensor (616).

12. The method (1300) of claim 11, wherein the first state comprises a deactivation state in which at least one of the wireless receiver or the wireless transmitter operates at a reduced power and the second state comprises an activation state in which at least one of the wireless receiver or the wireless transmitter (610) operates at operational power.

13. The method (1300) of claim 9, further comprising:
activating at least one illuminated indicator (602, 604) electrically connected to the at least one hardware processor (618, 624) to indicate a status of at least one of the wireless receiver (606, 608) or the wireless transmitter (610).

## Patentansprüche

1. Elektronische Vorrichtung (402, 600, 702, 704), umfassend:
einen drahtlosen Empfänger (606, 608), wobei der drahtlose Empfänger dazu ausgelegt ist, ein erstes drahtloses Protokoll zu verwenden und eine erste drahtlose Nachricht (406a, 407a, 408a, 409a) zu empfangen,
wobei die erste drahtlose Nachricht einen Identifikator enthält, der eine Vorrichtung (406, 407, 408, 409) identifiziert, die die erste drahtlose Nachricht gesendet hat;
einen drahtlosen Sender (610), wobei der drahtlose Sender dazu ausgelegt ist, ein zweites drahtloses Protokoll zu verwenden und eine zweite drahtlose Nachricht (412) an einen Echtzeit-Positionsdienste-, RTLS-, Server zu senden, wobei die zweite drahtlose Nachricht den Identifikator beinhaltet, wobei das erste drahtlose Protokoll und das zweite drahtlose Protokoll unterschiedliche drahtlose Protokolle sind; und
mindestens einen Hardware-Prozessor (618, 624), der dazu ausgelegt ist, eine empfangene Signalstärke-Angabe zu bestimmen, die eine Signalstärke eines drahtlosen Signals angibt, das zum Senden der ersten drahtlosen Nachricht verwendet wird;
**dadurch gekennzeichnet, dass** der Hardware-Prozessor (618, 624) ferner dazu ausgelegt ist, den drahtlosen Sender basierend auf dem drahtlosen Empfänger, der die erste drahtlose Nachricht empfangen hat, und basierend auf einem Vergleich der empfangenen Signalstärke-Angabe mit einem Signalstärke-Schwellenwert zu aktivieren.

2. Elektronische Vorrichtung (402, 600, 702, 704) nach Anspruch 1, wobei das erste drahtlose Protokoll Bluetooth beinhaltet und das zweite drahtlose Protokoll mindestens ein drahtloses Protokoll beinhaltet, das aus der Gruppe der 802.11-Protokolle des "Institute of Electrical and Electronics Engineers", IEEE, ausgewählt ist.

3. Elektronische Vorrichtung (402, 600, 702, 704) nach Anspruch 1, ferner umfassend: einen Bewegungssensor (616), der dazu ausgelegt ist, Bewegung zu erkennen, wobei der mindestens eine Hardware-Prozessor (618, 624) den drahtlosen Empfänger und/oder den drahtlosen Sender von einem ersten Zustand in einen zweiten Zustand überführt, basierend darauf, ob der Bewegungssensor Bewegung erkennt.

4. Elektronische Vorrichtung (402, 600, 702, 704) nach Anspruch 3, wobei der erste Zustand einen Deaktivierungszustand umfasst, in dem der drahtlose Empfänger und/oder der drahtlose Sender bei einer reduzierten Leistung arbeitet, und der zweite Zustand einen Aktivierungszustand umfasst, in dem der drahtlose Empfänger (606, 608) und/oder der drahtlose Sender (610) bei operativer Leistung arbeitet.

5. Elektronische Vorrichtung (402, 600, 702, 704) nach Anspruch 1, ferner umfassend mindestens zwei beleuchtete Anzeigen (602, 604), die durch den mindestens einen Hardware-Prozessor aktivierbar sind, wobei die mindestens zwei beleuchteten Anzeigen (538) einen Status des drahtlosen Empfängers und/oder des drahtlosen Senders angeben.

6. Elektronische Vorrichtung (402, 600, 702, 704) nach Anspruch 1, wobei:
der mindestens eine Prozessor (618, 624) elektrisch mit dem drahtlosen Sender (610) und dem drahtlosen Empfänger (606, 608) verbunden ist;
der mindestens eine Hardware-Prozessor, der drahtlose Sender und der drahtlose Empfänger in einem geschlossenen Gehäuse mit mindestens zwei Oberflächen untergebracht sind; und das Gehäuse mindestens einen flexiblen Clip (706) beinhaltet, der an mindestens einer der mindestens zwei Oberflächen befestigt ist, wobei das geschlossene Gehäuse unter Verwendung des mindestens einen flexiblen Clips an einem Kleidungsstück befestigt werden kann.

7. Ein Echtzeit-Positionsdienste-, RTLS-, System (400) umfassend:
mehrere Beacon-Tags (406, 407, 408, 409), die dazu ausgelegt sind, mindestens eine drahtlose Nachricht (406a, 407a, 408a, 409a) auszustrahlen, wobei die mindestens eine drahtlose Nachricht unter Verwendung eines ersten drahtlosen Protokolls ausgestrahlt wird; und
eine elektronische Vorrichtung (402, 600, 702, 704) nach einem der Ansprüche 1 bis 6.

8. System (400) nach Anspruch 7, wobei mindestens eines der mehreren Beacon-Tags (406, 407, 408, 409) dazu ausgelegt ist, die mindestens eine drahtlose Nachricht (406a, 407a, 408a, 409a) in einem festen Zeitintervall periodisch auszustrahlen.

9. Verfahren (1300), das von einer elektronischen Vorrichtung (402, 600, 702, 704) durchgeführt wird, umfassend:
Empfangen (1304) einer ersten drahtlosen Nachricht (406a, 407a, 408a, 409a) durch einen drahtlosen Empfänger (606, 608) der elektronischen Vorrichtung (402, 600, 702, 704) unter Verwendung eines ersten drahtlosen Protokolls,
wobei die erste drahtlose Nachricht einen Identifikator enthält, der eine Vorrichtung (406, 407, 408, 409) identifiziert, die die erste drahtlose Nachricht gesendet hat;
Bestimmen einer Angabe zur empfangenen Signalstärke, die eine Signalstärke eines drahtlosen Signals angibt, das zum Senden der ersten drahtlosen Nachricht verwendet wird;
Übertragen (1312) einer zweiten drahtlosen Nachricht (412) durch einen drahtlosen Sender (610) der elektronischen Vorrichtung (402, 600, 702, 704) an einen Echtzeit-Positionsdienste-, RTLS-, Server unter Verwendung eines zweiten drahtlosen Protokolls, wobei die zweite drahtlose Nachricht den Identifikator enthält, wobei das erste drahtlose Protokoll und das zweite drahtlose Protokoll unterschiedliche drahtlose Protokolle sind;
**dadurch gekennzeichnet, dass** das Verfahren ferner umfasst:
Aktivieren des drahtlosen Senders, basierend auf dem drahtlosen Empfänger, der die erste drahtlose Nachricht empfangen hat, und basierend auf einem Vergleich (1306) der empfangenen Signalstärke-Angabe mit einem Signalstärke-Schwellenwert, durch mindestens einen Hardware-Prozessor (618, 624) der elektronischen Vorrichtung (402, 600, 702, 704).

10. Verfahren (1300) nach Anspruch 9, wobei das erste drahtlose Protokoll Bluetooth beinhaltet und das zweite drahtlose Protokoll mindestens ein drahtloses Protokoll beinhaltet, das aus der Gruppe der 802.11-Protokolle des "Institute of Electrical and Electronics Engineers", IEEE, ausgewählt ist.

11. Verfahren (1300) nach Anspruch 9, ferner umfassend:
Erkennen von Bewegung durch einen Bewegungssensor (616) der elektronischen Vorrichtung (402, 600, 702, 704); und
Überführen des drahtlosen Empfängers (606, 608) und/oder des drahtlosen Senders (610) von einem ersten Zustand in einen zweiten Zustand, basierend auf der vom Bewegungssensor (616) erkannten Bewegung, durch den mindestens einen Hardware-Prozessor (618, 624).

12. Verfahren (1300) nach Anspruch 11, wobei der erste Zustand einen Deaktivierungszustand umfasst, in dem der drahtlose Empfänger und/oder der drahtlose Sender bei einer reduzierten Leistung arbeitet, und der zweite Zustand einen Aktivierungszustand umfasst, in dem der drahtlose Empfänger und/oder der drahtlose Sender (610) bei operativer Leistung arbeitet.

13. Verfahren (1300) nach Anspruch 9, ferner umfassend:
Aktivieren mindestens einer beleuchteten Anzeige (602, 604), die elektrisch mit dem mindestens einen Hardware-Prozessor (618, 624) verbunden ist, um einen Status des drahtlosen Empfängers (606, 608) und/oder des drahtlosen Senders (610) anzugeben.

## Revendications

1. Dispositif électronique (402, 600, 702, 704) comprenant :
un récepteur sans fil (606, 608), le récepteur sans fil étant configuré pour utiliser un premier protocole sans fil et recevoir un premier message sans fil (406a, 407a, 408a, 409a), le premier message sans fil comprenant un identificateur qui identifie un dispositif (406, 407, 408, 409) ayant envoyé le premier message sans fil;
un émetteur sans fil (610), l'émetteur sans fil étant configuré pour utiliser un second protocole sans fil et envoyer un second message sans fil (412) à un serveur de services de localisation en temps réel, RTLS, le second message sans fil comprenant l'identificateur, où le premier protocole sans fil et le second protocole sans fil sont des protocoles sans fil différents; et
au moins un processeur matériel (618, 624) configuré pour déterminer une indication d'intensité de signal reçu indiquant une intensité de signal d'un signal sans fil utilisé pour envoyer le premier message sans fil;
**caractérisé en ce que** le processeur matériel (618, 624) est en outre configuré pour activer l'émetteur sans fil sur la base du récepteur sans fil ayant reçu le premier message sans fil et sur la base d'une comparaison de l'indication d'intensité de signal reçu avec un seuil d'intensité de signal.

2. Dispositif électronique (402, 600, 702, 704) selon la revendication 1, dans lequel le premier protocole sans fil comprend le Bluetooth et le second protocole sans fil comprend au moins un protocole sans fil sélectionné dans le groupe de protocoles 802.11 de l'Institute of Electrical and Electronics Engineers, IEEE.

3. Dispositif électronique (402, 600, 702, 704) selon la revendication 1, comprenant en outre : un capteur de mouvement (616) configuré pour détecter un mouvement, dans lequel l'au moins un processeur matériel (618, 624) fait passer au moins l'un du récepteur sans fil ou de l'émetteur sans fil d'un premier état à un second état selon que le capteur de mouvement détecte un mouvement.

4. Dispositif électronique (402, 600, 702, 704) selon la revendication 3, dans lequel le premier état comprend un état de désactivation dans lequel au moins l'un du récepteur sans fil ou de l'émetteur sans fil fonctionne à une puissance réduite et le second état comprend un état d'activation dans lequel au moins l'un du récepteur sans fil (606, 608) ou de l'émetteur sans fil (610) fonctionne à une puissance opérationnelle.

5. Dispositif électronique (402, 600, 702, 704) selon la revendication 1, comprenant en outre au moins deux indicateurs lumineux (602, 604) pouvant être activés par l'au moins un processeur matériel, les au moins deux indicateurs lumineux (538) indiquant un état d'au moins l'un du récepteur sans fil ou de l'émetteur sans fil.

6. Dispositif électronique (402, 600, 702, 704) selon la revendication 1, dans lequel :
l'au moins un processeur (618, 624) est connecté électriquement à l'émetteur sans fil (610) et au récepteur sans fil (606, 608) ;
l'au moins un processeur matériel, l'émetteur sans fil et le récepteur sans fil sont logés dans un boîtier fermé ayant au moins deux surfaces; et le boîtier comprend au moins une pince flexible (706) fixée à au moins l'une des au moins deux surfaces, dans lequel le boîtier fermé peut être fixé à un article vestimentaire à l'aide de l'au moins une pince flexible.

7. Système de services de localisation en temps réel, RTLS, (400) comprenant :
une pluralité d'étiquettes de balises (406, 407, 408, 409), configurées pour diffuser au moins un message sans fil (406a, 407a, 408a, 409a), l'au moins un message sans fil étant diffusé en utilisant un premier protocole sans fil ; et
un dispositif électronique (402, 600, 702, 704) selon l'une quelconque des revendications 1 à 6.

8. Système (400) selon la revendication 7, dans lequel au moins l'une de la pluralité d'étiquettes de balises (406, 407, 408, 409) est configurée pour diffuser périodiquement l'au moins un message sans fil (406a, 407a, 408a, 409a) à un intervalle de temps fixe.

9. Procédé (1300) exécuté par un dispositif électronique (402, 600, 702, 704), comprenant :
la réception (1304) d'un premier message sans fil (406a, 407a, 408a, 409a) par un récepteur sans fil (606, 608) du dispositif électronique (402, 600, 702, 704) en utilisant un premier protocole sans fil, le premier message sans fil comprenant un identificateur qui identifie un dispositif (406, 407, 408, 409) ayant envoyé le premier message sans fil;
la détermination d'une indication d'intensité de signal reçu indiquant une intensité de signal d'un signal sans fil utilisé pour envoyer le premier message sans fil ;
la transmission (1312) d'un second message sans fil (412) par un émetteur sans fil (610) du dispositif électronique (402, 600, 702, 704) à un serveur de services de localisation en temps réel, RTLS, en utilisant un second protocole sans fil, le second message sans fil comprenant l'identificateur, où le premier protocole sans fil et le second protocole sans fil sont des protocoles sans fil différents;
**caractérisé en ce que** le procédé comprend en outre :
l'activation, par au moins un processeur matériel (618, 624) du dispositif électronique (402, 600, 702, 704), de l'émetteur sans fil sur la base du récepteur sans fil ayant reçu le premier message sans fil et sur la base d'une comparaison (1306) de l'indication d'intensité de signal reçu avec un seuil d'intensité de signal.

10. Procédé (1300) selon la revendication 9, dans lequel le premier protocole sans fil comprend le Bluetooth et le second protocole sans fil comprend au moins un protocole sans fil sélectionné dans le groupe de protocoles 802.11 de l'Institute of Electrical and Electronics Engineers, IEEE.

11. Procédé (1300) selon la revendication 9, comprenant en outre :
la détection d'un mouvement par un capteur de mouvement (616) du dispositif électronique (402, 600, 702, 704) ; et
le passage, par l'au moins un processeur matériel (618, 624), d'au moins l'un du récepteur sans fil (606, 608) ou de l'émetteur sans fil (610) d'un premier état à un second état sur la base du mouvement détecté par le capteur de mouvement (616).

12. Procédé (1300) selon la revendication 11, dans lequel le premier état comprend un état de désactivation dans lequel au moins l'un du récepteur sans fil ou de l'émetteur sans fil fonctionne à une puissance réduite et le second état comprend un état d'activation dans lequel au moins l'un du récepteur sans fil ou de l'émetteur sans fil (610) fonctionne à une puissance opérationnelle.

13. Procédé (1300) selon la revendication 9, comprenant en outre :
l'activation d'au moins un indicateur lumineux (602, 604) connecté électriquement à l'au moins un processeur matériel (618, 624) pour indiquer un état d'au moins l'un du récepteur sans fil (606, 608) ou de l'émetteur sans fil (610).
